# EUROPEAN PATENT APPLICATION

(11) **EP 3 124 606 A1**
(43) Date of publication of application: **01.02.2017**
(21) Application number: 14887202.1
(22) Date of filing: 27.12.2014
(51) Int. Cl.: C12N 15/00, C12M 1/00, C12N 15/115, G01N 33/53, G01N 33/566

(54) **RNA APTAMER AND SENSOR USING SAME**

(30) Priority: 28.03.2014 JP 2014068659
(71) Applicant: Kyocera Corporation, Kyoto-shi, Kyoto 612-8501 (JP)
(72) Inventor: KURIOKA, Hideharu, Kyoto-shi Kyoto 612-8501 (JP); TANAKA, Hiroyasu, Kyoto-shi Kyoto 612-8501 (JP)
(74) Representative: Viering, Jentschura & Partner mbB Patent- und Rechtsanwälte
(86) International application number: PCT/JP2014/084719
(87) International publication number: WO 2015/145916

(57) **Abstract**

An RNA aptamer (130) is provided with a sequence (S) of linkage order of UAUUAGGACCA.

## Description

### Technical Field

The present invention relates to: an RNA aptamer capable of measuring a property of an analyte liquid or a component contained in the analyte liquid; and a sensor employing the same.

### Background Art

Known are: an antibody employed for measurement of hemoglobin contained in an analyte liquid performed by using a sensing element; and a sensor employing the same (for example, see Patent Literature 1 or 2).

However, although Patent Literatures 1 and 2 disclose an antibody and a sensor used for measuring hemoglobin contained in an analyte liquid, no aptamer is disclosed therein.

Here, the antibody employed for the measurement is produced by using animal cells. Thus, batch-to-batch variation is caused by individual specificity and, further, the production of such an antibody is not suitable for mass production by chemical synthesis and hence reduction of the production cost is also difficult. Further, the antibody is a protein and hence has a property of being easily denatured under a hot or dry environment and hence not satisfactorily preservable.

Thus, an aptamer has been desired that can specifically bind to hemoglobin and, further, has small quality fluctuation, a low cost, and excellent preservability.

### Citation List

### Patent Literature

Patent Literature 1: Japanese Unexamined Patent Publication JP-A 2002-209579
Patent Literature 2: Japanese Unexamined Patent Publication JP-A 06-113830 (1994)

### Summary of Invention

### Technical Problem

An aptamer specifically binding to hemoglobin and a sensor employing the same have been desired.

### Solution to Problem

An RNA aptamer according to an embodiment of the invention includes a sequence of linkage order of UAUUAGGACCA. Here, an A denotes a nucleotide whose base is adenine, a G denotes a nucleotide whose base is guanine, a C denotes a nucleotide whose base is cytosine, and a U denotes a nucleotide whose base is uracil. When such a configuration is provided, the RNA aptamer can effectively and specifically bind to at least one kind of fractionation components of hemoglobin (also referred to as Hb, hereinafter).

A sensor according to an embodiment of the invention includes: a substrate; and the above-mentioned RNA aptamer immobilized to the substrate. When such a configuration is provided, the RNA aptamer can effectively and specifically bind to at least one kind of fractionation components of hemoglobin.

### Brief Description of Drawings

FIG. 1 is a diagram showing an RNA aptamer according to an embodiment of the invention;
FIG. 2 is a diagram showing an sensor according to an embodiment of the invention, wherein FIG. 2(a) is a plan view, FIG. 2(b) is a sectional view in the length direction, and FIG. 2(c) is a sectional view in the width direction;
FIG. 3 is an enlarged sectional view showing a part of the sensor of FIG. 2;
FIG. 4 is a plan view showing a sensing element of a sensor of FIG. 2;
FIG. 5 is an exploded plan view of a sensor of FIG. 2;
FIG. 6 is a plan view showing a production process of the sensor of FIG. 2;
FIG. 7 shows plan views of modified examples of the sensor of FIG. 2, wherein FIGS. 7(a) and 7(b) are views corresponding to FIG. 6(d), and FIG. 7(c) is a view corresponding to FIG. 2(a);
FIG. 8 is a diagram showing a modified example of the sensor of FIG. 2, wherein FIG. 8(a) is a plan view, FIG. 8(b) is a sectional view in the length direction, and FIG. 8(c) is a sectional view in the width direction;
FIG. 9 is a plan view showing a production process of the sensor of FIG. 8;
FIG. 10 is a diagram showing a modified example of the sensor of FIG. 2, in which a production process thereof is shown in particular;
FIG. 11 is a plan view showing a modified example of the sensor of FIG. 2, which corresponds to FIG. 6(d);
FIG. 12 is a side view showing an example of a detection part of a sensor of FIG. 2;
FIG. 13 is a side view showing an example of a detection part of a sensor of FIG. 2;
FIG. 14 is a diagram showing experimental data concerning a sensor (an RNA aptamer) according to an embodiment of the invention;
FIG. 15 is a diagram showing experimental data concerning a sensor (an RNA aptamer) according to an embodiment of the invention; and
FIG. 16 is a diagram showing experimental data concerning a sensor (an RNA aptamer) according to an embodiment of the invention.

### Description of Embodiments

An RNA aptamer according to an embodiment of the invention and an embodiment of a sensor employing the same are described below in detail with reference to the drawings and the like. Here, in the individual drawings described below, like component members are designated by like reference numerals. Further, the size of each member, the distance between members, and the like shown in the figures are merely schematic and may be different from actual ones in some cases.

### <RNA aptamer>

An RNA aptamer 130 according to an embodiment of the invention includes a sequence S of linkage order of UAUUAGGACCA. Here, an A denotes a nucleotide whose base is adenine, a G denotes a nucleotide whose base is guanine, a C denotes a nucleotide whose base is cytosine, and a U denotes a nucleotide whose base is uracil (this notation is employed also in the following description). When such a sequence S is provided, the RNA aptamer 130 can effectively and specifically bind to at least one kind of fractionation components of Hb. Specifically, the RNA aptamer 130 according to the present embodiment can specifically bind to at least HbA0 among the fractionation components of Hb. Here, the RNA aptamer 130 according to the present embodiment has a tendency not to bind to human serum albumin (HSA) and human IgG (h-IgG).

Here, it is preferable that in at least one group among the C's and the U's contained in the RNA aptamer 130, a fluorine group is provided at the 2'-position of the sugar constituting each nucleotide. By virtue of this, the structural stability of the RNA aptamer 130 can be improved. In the present embodiment, in both the C's and the U's, a fluorine group is provided at the 2'-position of the sugar constituting each nucleotide.

An example of the RNA aptamer 130 according to the present embodiment includes: a first region 130a containing a U-base and an A-base located at both ends of the sequence S; and a second region 130b having a linkage order of AUUAGGACC in a direction from the above-mentioned U to the above-mentioned A. Further, the RNA aptamer 130 includes a terminal region 130c provided with a 3'-terminal 130c1 and a 5'-terminal 130c2.

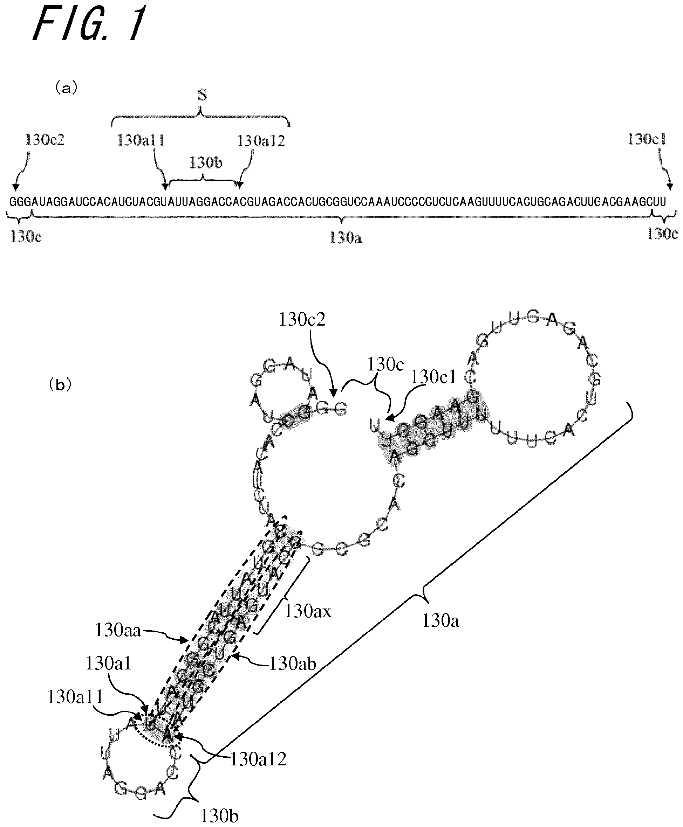

Here, according to software (software name: CentroidFold) for predicting the secondary structure, an example of the RNA aptamer 130 according to the present embodiment has a shape shown in FIG. 1(b). First, a first base pair 130a constructed from the U-base and the A-base in the first region 130a and a site having a linkage order of AUUAGGACC in the direction from the U to the A constituting the first base pair 130a in the second region 130b form a loop shape. Further, in the first region 130a, at least one base pair is formed by a plurality of nucleotides in a site continuous to the first base pair 130a consisting of the U-base and the A-base so that a stem shape is formed. That is, the RNA aptamer 130 according to the present embodiment has a so-called stem loop shape.

### (First region)

As described above, the first region 130a includes a U-base and an A-base.

Further, the first region 130a may further include a plurality of nucleotides attached to at least one of the U and the A located at both ends of the sequence S. By virtue of this, immobilization to any other member can easily be achieved. For example, the plurality of nucleotides may be constructed so as to include: a first sequence 130aa composed of at least one nucleotide attached to the U located at a first end 130a11 of the sequence S; and a second sequence 130ab composed of at least one nucleotide attached to the A located at a second end 130a12 of the sequence S.

Further, the first region 130a may include a first site 130ax where the first sequence 130aa and the second sequence 130ab constitute at least one base pair. By virtue of this, the structure of the RNA aptamer 130 itself can be stabilized by the base pair. Here, the first region 130a may include a plurality of base pairs. Then, the plurality of base pairs may be consecutive to each other.

Further, the first region 130a may include a second site 130ay where the first sequence 130aa and the second sequence 130ab do not constitute a base pair. By virtue of this, flexibility is provided so that effective orientation can be achieved at the time of immobilization. Here, the first region 130a may include a plurality of sites where no base pair is formed. Then, these sites where no base pair is formed may be consecutive to each other.

Further, it is preferable that the U located at the first end 130a11 of the sequence S is positioned on the 5'-terminal 130c2 side relative to the A located at the second end 130a12 of the sequence S. By virtue of this, specific binding to Hb is easily generated.

### (Second region)

As described above, in the second region 130b, nucleotides are attached together in the order of AUUAGGACC in the direction from the above-mentioned U to the above-mentioned A in the first region 130a. When such a configuration is provided, it is expected that the spatial configuration of the second region 130b and the spatial configuration of hemoglobin serving as a detection target interact with each other so that the RNA aptamer and hemoglobin specifically bind to each other and hence hemoglobin can effectively be detected.

### (Terminal region)

The terminal region 130c may contain a modification group such as a biotin, a thiol group, and an amino group. The terminal region 130c indicates an end part of the RNA aptamer 130 and consists of the 3'-terminal 130c1 and the 5'-terminal 130c2. By virtue of this, immobilization of the RNA aptamer 130 to any other member can easily be achieved. Here, each of the above-mentioned modification groups may be attached to at least one of the 3'-terminal 130c1 and the 5'-terminal 130c2 in the terminal region 130c. For example, when the above-mentioned modification groups are attached to both ends, variation of immobilization can be increased or, alternatively, binding strength of the immobilization can be improved.

Here, when the terminal region 130c includes a biotin, it is preferable that a streptavidin (referred to as an SA in some cases, hereinafter) is bound to this biotin. According to this, immobilization to any other member becomes easy by virtue of the amide linkage between the biotin and the streptavidin.

Further, when the terminal region 130c includes a thiol group, the RNA aptamer 130 can be attached to gold by gold-thiol linkage. Thus, with the intervention of the gold attached to the thiol group, immobilization to any other member can relatively easily be achieved. Here, when a thiol group is provided in the terminal region 130c, a spacer having a predetermined length may intervene between the thiol group and the RNA aptamer 130. By virtue of this, when the height from the substrate to the RNA aptamer 130 is adjusted suitably, the detection sensitivity for a detection target by the RNA aptamer 130 can be improved.

Further, when the terminal region 130c includes an amino group, it is preferable that a first substance is included that forms an amide linkage with the amino group. Here, for example, polyethylene glycol (PEG) may be employed as the first substance. At that time, the amino group forms an amide linkage with a carboxyl group modified to a terminal of the polyethylene glycol. Then, with the intervention of a thiol group modified to the other terminal of the polyethylene glycol, immobilization to any other member can be achieved.

### <Sensor>

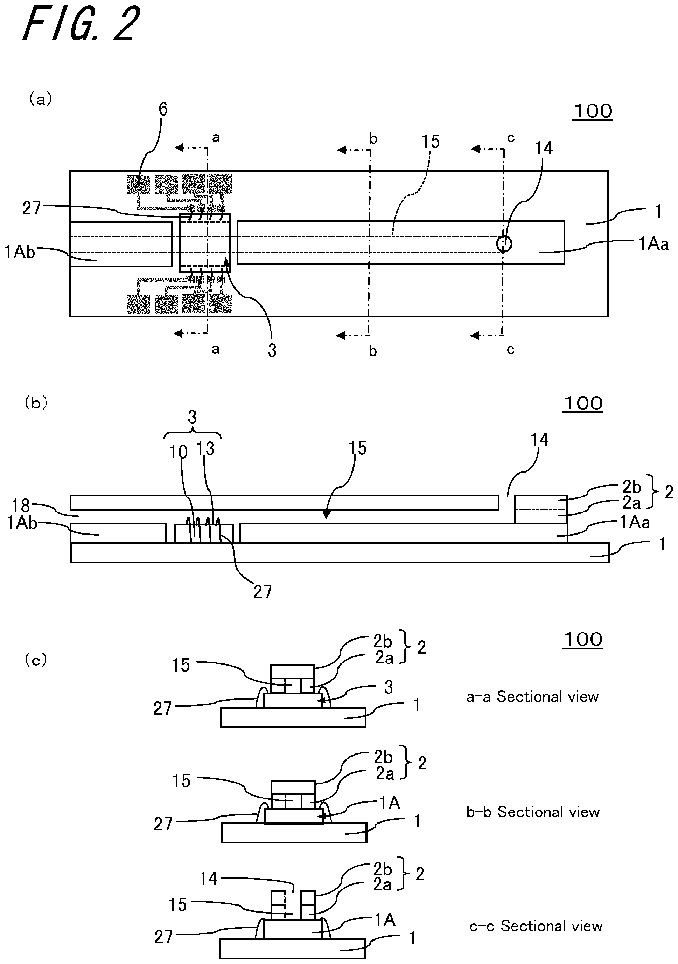

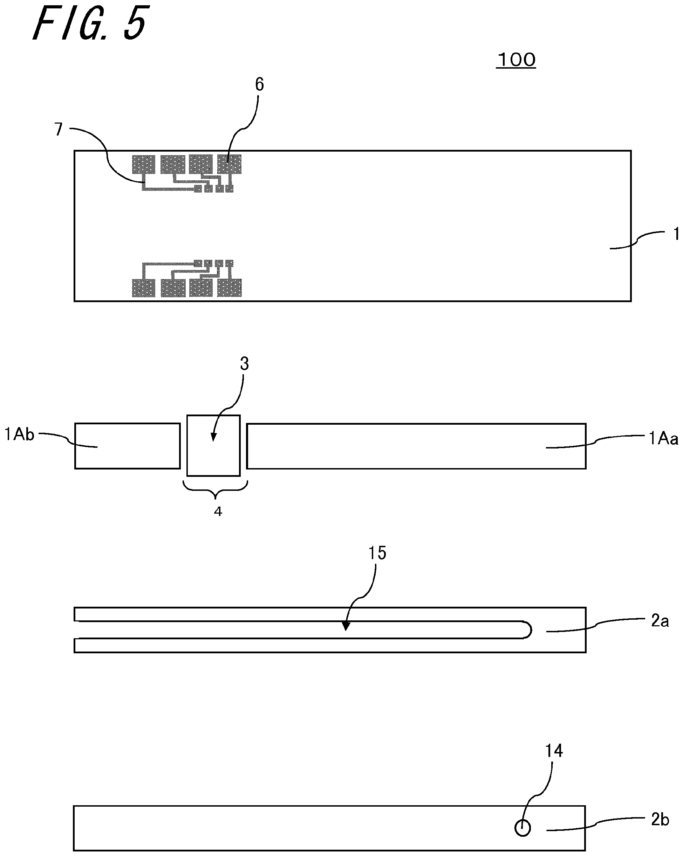

A sensor 100 according to an embodiment of the invention is described below with reference to FIGS. 2 to 6.

As shown in FIG. 2, the sensor 100 according to the present embodiment includes mainly a first cover member 1, an intermediate cover member 1A, a second cover member 2, and a sensing element 3.

Specifically, as shown in FIG. 2(b), the sensor 100 includes: an inflow part 14 into which an analyte liquid flows; and a passage 15 which is continuous to the inflow part 14, is surrounded by the intermediate cover member 1A and the second cover member 2, and extends at least to the detection part 13. FIG. 2(c) provides sectional views of FIG. 2(a) and shows an a-a sectional view, a b-b sectional view, and a c-c sectional view in the order from top to bottom. As shown in FIG. 2(b), the inflow part 14 is located on side faces of the intermediate cover member 1A and the second cover member 2. Here, as shown in FIG. 2(b), the inflow part 14 may penetrate through the second cover member 2 in the thickness direction.

In the sensor 100 according to the present embodiment, the sensing element and the intermediate cover member constituting at least a part of the passage are provided in an aligned manner on an upper face of the first cover member. Thus, even when a sensing element having a non-negligible thickness is employed, a passage for analyte liquid extending from the inflow part to the detection part can be ensured so that an analyte liquid suctioned through the inflow part by a capillary phenomenon or the like can be led to the detection part. That is, even when a sensing element having a non-negligible thickness is employed, it is possible to provide a sensor in which a suction mechanism for analyte liquid is provided in itself and measuring work is simplified. Further, in the passage for analyte liquid, contact angles θ1a and θ2a of the member surfaces located in the upstream of the sensing element relative to the analyte liquid are smaller than a contact angle θ3 of the surface of the sensing element relative to the analyte liquid. Thus, the analyte liquid having flowed through the inflow part can smoothly be led toward the sensing element (the detection part) along the member surfaces located in the upstream.

### (First cover member 1)

As shown in FIG. 2(b), the first cover member 1 has a flat plate shape. For example, the thickness thereof is 0.1 to 0.5 mm. The planar shape of the first cover member 1 is substantially rectangular. For example, the length of the first cover member 1 in the length direction is 1 to 5 cm. For example, the length in the width direction is 1 to 3 cm. As the material of the first cover member 11, for example, paper, plastics, celluloid, ceramics, nonwoven fabric, glass, or the like may be employed. From the perspective of a required strength and a cost, plastics are preferable.

Further, as shown in FIG. 2(a), terminals 6 and wirings 7 leading from the terminals 6 to the vicinity of the sensing element 3 are formed on the upper face of the first cover member 1. The terminals 6 are formed on both sides of the sensing element 3 in the width direction on an upper face of the intermediate cover member 1A. When measurement using the sensor 100 is performed by an external measuring instrument (not shown), the terminals 6 are electrically connected to the external measuring instrument. Further, the terminals 6 and the sensing element 3 are electrically connected through the wirings 7 and the like. Then, a signal from the external measuring instrument is inputted through the terminal 6 to the sensor 100 and then a signal from the sensor 100 is outputted through the terminal 6 to the external measuring instrument.

### (Intermediate cover member 1A)

In the present embodiment, as shown in FIG. 2(a), the intermediate cover member 1A is located on the upper face of the first cover member 1 along the sensing element 3. Further, the intermediate cover member 1A and the sensing element 3 are located with a gap in between.

The intermediate cover member 1A has a flat-plate frame shape in which a recess forming site 4 is formed in a flat-plate shaped plate. For example, the thickness thereof is 0.1 mm to 0.5 mm.

In the present embodiment, as shown in FIG. 2(a), the recess forming site 4 is a site for dividing a first upstream part 1Aa from a first downstream part 1Ab. When the intermediate cover member 1A provided with the recess forming site 4 is joined to the first cover member 1 having a flat plate shape, the first cover member 1 and the intermediate cover member 1A form an element accommodation recess 5. That is, the upper face of the first cover member 1 located on an inner side of the recess forming site 4 forms a bottom face of the element accommodation recess 5 and an inner wall of the recess forming site 4 forms an inner wall of the element accommodation recess 5.

For example, resin (including plastics), paper, nonwoven fabric, or glass may be employed as the material of the intermediate cover member 1A. More specifically, it is preferable that a resin material such as a polyester resin, a polyethylene resin, an acrylic resin, and a silicone resin is employed. Here, the material of the first cover member 1 and the material of the intermediate cover member 1A may be different from each other.

Further, in the present embodiment, the intermediate cover member 1A includes the first upstream part 1Aa and the first downstream part 1Ab. Then, as shown in FIG. 2(a), when viewed in a top view, the sensing element 3 is located between the first upstream part 1Aa and the first downstream part 1Ab. By virtue of this, in the analyte liquid flowing on the sensing element 3 through the first upstream part 1Aa along the passage 15, an amount exceeding the amount required for measurement flows to the first downstream part 1Ab side. Thus, an appropriate amount of the analyte liquid can be supplied to the sensing element 3.

Here, it is preferable that the thickness of the intermediate cover member 1A is greater than the thickness of the sensing element 3.

### (Second cover member 2)

As shown in FIG. 2(b), the second cover member 2 covers at least a part of the sensing element 3 and is joined to the intermediate cover member 1A. For example, resin (including plastics), paper, nonwoven fabric, or glass may be employed as the material of the second cover member 2. More specifically, it is preferable that a resin material such as a polyester resin, a polyethylene resin, an acrylic resin, and a silicone resin is employed. Here, the material of the first cover member 1 and the material of the second cover member 2 may be the same as each other. By virtue of this, it is possible to suppress deformation caused by a difference in the thermal expansion coefficients of these members. Here, as for the configuration of the second cover member 2, the second cover member 2 may be joined only to the intermediate cover member 1A or, alternatively, may be joined to both the first cover member 1 and the intermediate cover member 1A.

Here, the second cover member 2 includes a third substrate 2a and a fourth substrate 2b.

The third substrate 2a is glued to the upper face of the intermediate cover member 1A. The third substrate 2a has a flat plate shape and the thickness thereof is, for example, 0.1 mm to 0.5 mm. The fourth substrate 2b is glued to an upper face of the third substrate 2a. The fourth substrate 2b has a flat plate shape and the thickness thereof is, for example, 0.1 mm to 0.5 mm. Then, when the fourth substrate 2b is joined to the third substrate 2a, as shown in FIG. 2(b), the passage 15 is formed in a lower face of the second cover member 2. The passage 15 extends from the inflow part 14 to at least a region immediately above the detection part 13 and, for example, has a cross section of rectangular shape.

In the present embodiment, as shown in FIG. 2(b), at an end of the passage 15, the third substrate 2a does not exist and hence a gap between the fourth substrate 2b and the intermediate cover member 1A serves as an air release hole 18. The air release hole 18 is used for releasing the air and the like in the passage 15 to the outside. The air release hole 18 may have a whatever shape such as a cylindrical shape and a quadrangular prism shape as long as the air in the passage 15 can be released. However, when an aperture of the air release hole 18 is excessively large, an area of contact of the analyte liquid present in the passage 15 with an outside air increases and hence the water in the analyte liquid easily evaporates. In this situation, concentration change is easily caused in the analyte liquid and hence an measurement accuracy is degraded. Thus, it is preferable that the aperture of the air release hole 18 is set to be not excessively large to an extent exceeding the necessity. Specifically, in a case where the air release hole 18 has a cylindrical shape, the diameter thereof is set to be 1 mm or smaller and, in a case where the air release hole 18 has a quadrangular prism shape, one side thereof is set to be 1 mm or smaller. Further, an inner wall of the air release hole 18 has hydrophobicity. By virtue of this, a situation is suppressed that the analyte liquid filling the passage 15 leaks through the air release hole 18 to the outside.

The first cover member 1, the intermediate cover member 1A, and the second cover member 2 may be all formed of the same material. By virtue of this, the thermal expansion coefficients of the individual members are substantially equal to each other and hence deformation caused by a difference in the thermal expansion coefficients of the individual members is suppressed. Further, in some cases, a biomechanical material is applied to the detection part 13 and then some of these materials easily deteriorate owing to external light such as ultraviolet light. In such cases, it is sufficient that a non-transparent material having a light shielding property is employed as the materials of the first cover member 1, the intermediate cover member 1A, and the second cover member 2. On the other hand, in a case where deterioration is hardly caused by external light of the detection part 13, the second cover member 2 constituting the passage 15 may be formed from an almost transparent material. In this case, the situation of the analyte liquid flowing through the inside of the passage 15 can visually be recognized.

### (Sensing element 3)

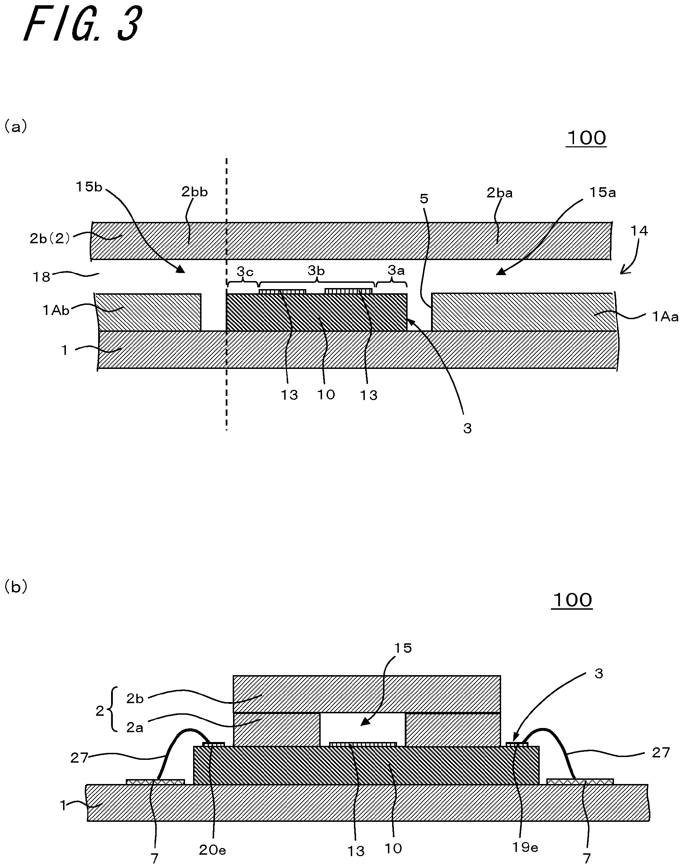

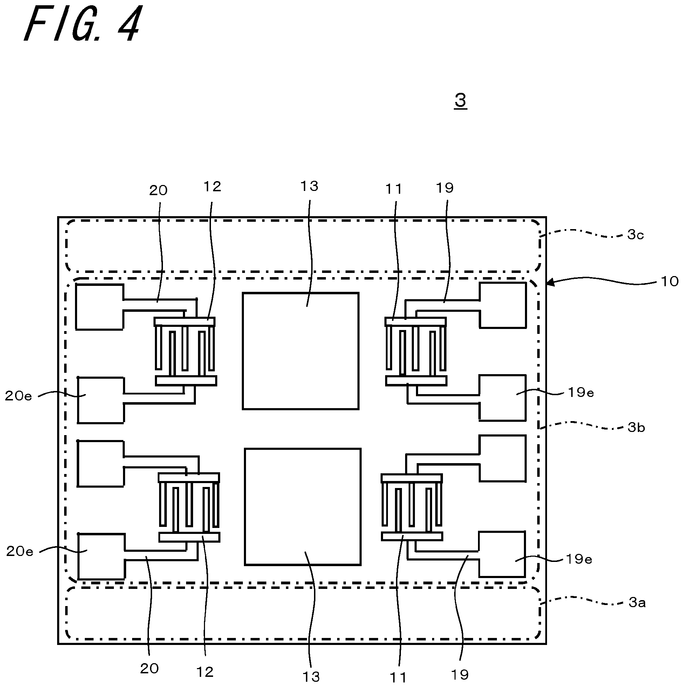

As shown in FIG. 2(b), the sensing element 3 includes: a substrate 10 located on the upper face of the first cover member 1; and at least one detection part 13 which is located on an upper face of the substrate 10 and detects a detection target contained in the analyte liquid. Details of the sensing element 3 are shown in FIGS. 3(b) and 4.

Here, in the present embodiment, as shown in FIG. 4, an electrode pattern is provided on the upper face of the substrate 10. Then, when necessary, an insulating member 28 may be provided so as to cover the electrode pattern. Here, in a case where a SAW element is employed as the sensing element 3, IDT (InterDigital Transducer) electrodes correspond to the electrode pattern. In the present embodiment, a first IDT electrode 11, a second IDT electrode 12, a first extraction electrode 19, a second extraction electrode 20, and the like described later are provided on the upper face of the substrate 10. In the present embodiment, as shown in FIG. 3(b), for example, the second cover member 2 is fixed to the upper face of the substrate 10 over the IDT electrodes 11 and 12.

### (Substrate 10)

For example, the substrate 10 is composed of a substrate constructed from a single crystal having piezoelectricity like a lithium tantalate (LiTaO3) single crystal, a lithium niobate (LiNbO3) single crystal, and quartz. The planar shape and the individual dimensions of the substrate 10 may be set up suitably. As an example, the thickness of the substrate 10 is 0.3 mm to 1 mm.

### (IDT electrodes 11 and 12)

As shown in FIG. 4, the first IDT electrode 11 includes one pair of comb electrodes. The comb electrodes are constructed from: two bus bars opposing each other; and a plurality of electrode fingers extending from one bus bar to the other bus bar side. Then, the one pair of comb electrodes is disposed so that the plurality of electrode fingers engage with each other. The second IDT electrode 12 is also constructed similarly to the first IDT electrode 11. The first IDT electrode 11 and the second IDT electrode 12 constitute IDT electrodes of transversal type.

The first IDT electrode 11 is used for generating a predetermined surface acoustic wave (SAW). Then, the second IDT electrode 12 receives the SAW generated by the first IDT electrode 11. In order to allow the second IDT electrode 12 to receive the SAW generated by the first IDT electrode 11, the first IDT electrode 11 and the second IDT electrode are aligned on the same straight line. The frequency characteristics can be designed with adopting as parameters the number of electrode fingers of the first IDT electrode 11 and the second IDT electrode 12, the distance between adjacent electrode fingers, the crossing width of the electrode fingers, and the like. The SAW excited by the IDT electrode may be of various oscillation modes. However, in the sensing element 3 according to the present embodiment, for example, a traverse oscillation mode referred to as an SH wave is employed.

Here, an elastic member for suppressing the SAW reflection may be provided on an outer side of the first IDT electrode 11 and the second IDT electrode 12 in a propagation direction (width direction) of the SAW. For example, the frequency of the SAW may be set within a range from several megahertz (MHz) to several gigahertz (GHz). Among these, a value from a few hundred MHz to 2 GHz is practical and, further, size reduction of the sensing element 3 as well as size reduction of the sensor 100 can be achieved.

### (Extraction electrodes 19 and 20)

As shown in FIG. 4, the first extraction electrode 19 is connected to the first IDT electrode 11 and the second extraction electrode 20 is connected to the second IDT electrode 12. The first extraction electrode 19 is extracted from the first IDT electrode 11 on a side opposite to the detection part 13 and then an end part 19e of the first extraction electrode 19 is electrically connected to the wiring 7 provided on the first cover member 1. The second extraction electrode 20 is extracted from the second IDT electrode 12 on a side opposite to the detection part 13 and then an end part 20e of the second extraction electrode 20 is electrically connected to the wiring 7.

Here, for example, the first IDT electrode 11, the second IDT electrode 12, the first extraction electrode 19, and the second extraction electrode 20 are formed of aluminum, an alloy of aluminum and copper, or the like. Further, these electrodes may have a multilayer structure. When a multilayer structure is employed, for example, the first layer may contain titanium or chromium and the second layer may contain aluminum or an aluminum alloy.

### (Detection part 13)

As shown in FIG. 4, the detection part 13 is located on the surface of the substrate 10 and positioned on the propagation path of the acoustic wave propagating from the first IDT electrode 11 to the second IDT electrode 12. The following description is given for an example that the RNA aptamer 130 according to the present embodiment mentioned above is employed.

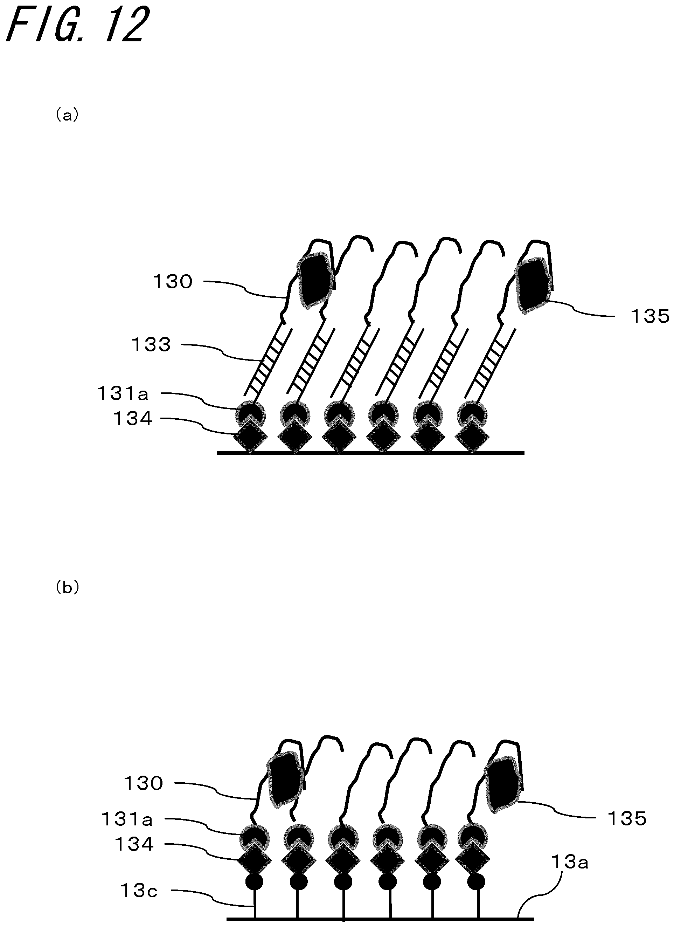

For example, as shown in FIG. 12(a), the detection part 13 includes: a plurality of SA's 134 fixed to the substrate 10; a plurality of biotins 131a individually attached to the plurality of SA's 134; and a plurality of RNA aptamers (binding parts) 130 each attached to each of the plurality of biotins 131a with the intervention of a double strand 133. That is, in the RNA aptamer (the binding part) 130, the biotin 131a is provided in the terminal region 130c with the intervention of the double strand 133. Then, the double strand 133 has a configuration that AAAAAAAAAAAAAAAAAA attached to the 3'-terminal of the RNA aptamer 130 and 5'-TTTTTTTTTTTTTTTTTT attached to the biotin 131a form base pairs.

Here, as a modified example of the detection part 13, a configuration shown in FIG. 12(b) may be employed.

In the present modified example, the detection part 13 may include: an immobilization membrane 13a located on the surface of the substrate 10; a chain-like substance (SAM: Self-Assembled Monolayer) 13c attached to the immobilization membrane 13a; a plurality of SA's 134 attached to the SAM; a plurality of biotins 131a individually attached to the plurality of SA's 134; and a plurality of RNA aptamers (binding parts) 130 individually attached to the plurality of biotins 131a. Here, as the substance forming the immobilization membrane 13a, for example, Au (gold), Ti, Cu, or the like may be employed. However, Au is preferable. Here, for example, the immobilization membrane 13a may be composed of a gold film or may have a two-layer structure that a gold film is formed on a chromium film. Further, a protective film 13b may intervene between the substrate 10 and the immobilization membrane 13a. Further, as the chain-like substance 13c, for example, alkane, polyethylene glycol, or a complex molecule formed between alkane and polyethylene glycol may be employed.

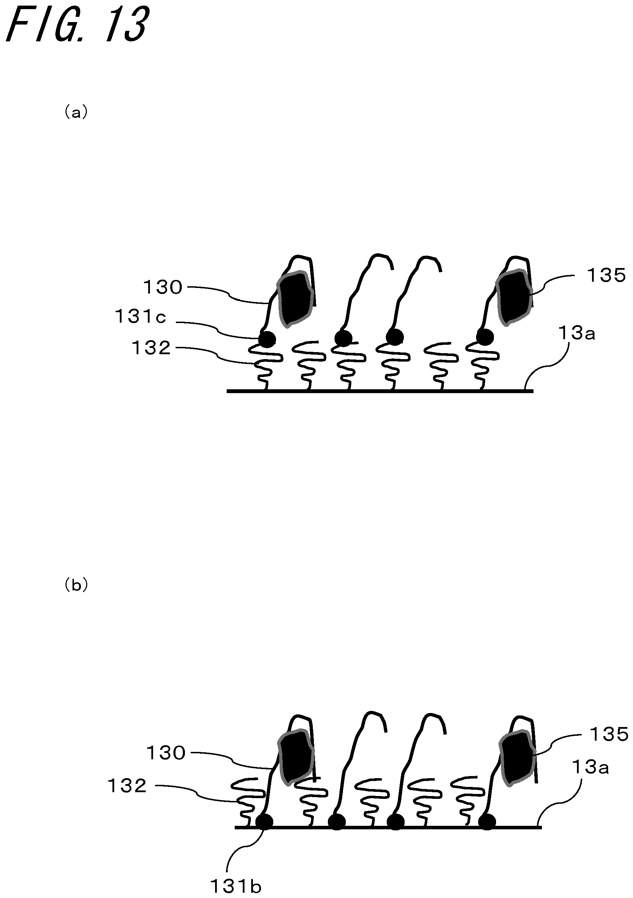

Further, as a modified example of the detection part 13, a configuration shown in FIG. 13(a) may be employed.

In the present modified example, in the RNA aptamer 130 of the detection part 13, the terminal region 130c thereof includes an amino group. In this case, a first substance 132 (the chain-like substance 13c) which forms an amide linkage with the amino group 131c may be included. Here, for example, polyethylene glycol (PEG) may be employed as the first substance 132. At that time, the amino group 131c forms an amide linkage with a carboxyl group modified to a terminal of the polyethylene glycol. Then, with the intervention of a thiol group 131b modified to the other terminal of the polyethylene glycol, immobilization to any other member can be achieved. Specifically, as shown in FIG. 13(a), the detection part 13 may include: an immobilization membrane (Au) 13a located on the surface of the substrate 10; a plurality of chain-like substances (polyethylene glycol) 13c attached to the immobilization membrane 13a; and a plurality of RNA aptamers (binding parts) 130 each attached to each of the plurality of chain-like substances 13c with the intervention of an amino group 131c. Then, active etherification may be performed on the carboxyl group located at a terminal of the polyethylene glycol so that linkage with the RNA aptamer 130 is established.

Further, as a modified example of the detection part 13, a configuration shown in FIG. 13(b) may be employed.

In the present modified example, in the RNA aptamer 130 of the detection part 13, the terminal region 130c thereof includes a thiol group 131b. In this case, the RNA aptamer 130 can be attached to gold by gold-thiol linkage. Thus, with the intervention of the gold attached to the thiol group 131b, immobilization to any other member can be relatively easily achieved. Specifically, as shown in FIG. 13(b), the detection part 13 may include: an immobilization membrane (Au) 13a located on the surface of the substrate 10; and a plurality of chain-like substances (polyethylene glycol) 13c and a plurality of RNA aptamers (binding parts) 130 individually attached to the immobilization membrane 13a.

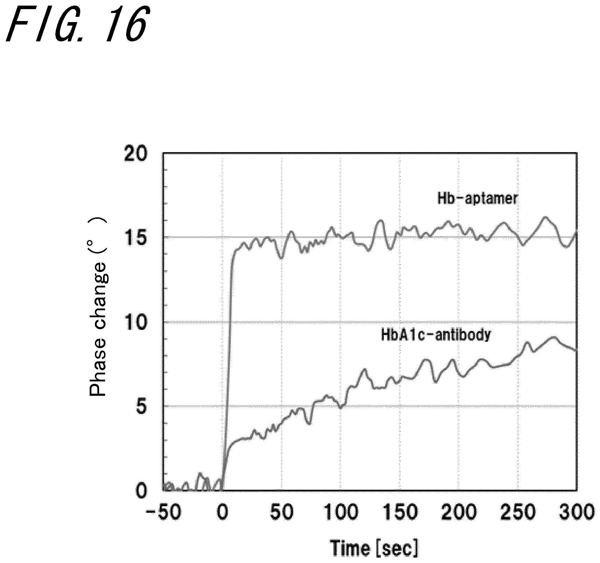

Here, when the first IDT electrode, the second IDT electrode, and the detection part 13 which are disposed along the width direction of the sensor 100 are regarded as one set, as shown in FIG. 4, the sensor 100 according to the present embodiment includes two sets. By virtue of this, when the detection target expected to react in one detection part 13 is set so as to be different from the detection target expected to react in the other detection part 13, two kinds of detection targets can be detected by using one sensor. For example, the RNA aptamer 130 capable of specifically binding to Hb may be provided in one detection part 13, and an antibody capable of specifically binding to HbA1c may be provided in the other detection part 13. As such, when an aptamer and an antibody used for different detection targets from each other are employed as a set, a plurality of detection targets can simultaneously be detected. For example, as shown in FIG. 16, by using data obtained by simultaneous measurement of Hb and HbA1c, the fraction of HbA1c in Hb can be calculated. Further, in one of the two sets, a reference electrode may be provided in place of the detection part 13 so that a reference part may be constructed.

### (Detection of detection target by using sensing element 3)

When detection is to be performed on an analyte liquid in the sensing element 3 employing a SAW, first, a predetermined voltage is applied from an external measuring instrument through the wiring 7, the first extraction electrode 19, and the like onto the first IDT electrode 11. Then, oscillation of the surface of the substrate 10 is excited in the region where the first IDT electrode 11 is formed, so that a SAW having a predetermined frequency is generated. A part of the generated SAW propagates toward the detection part 13, then passes through the detection part 13 and then reaches the second IDT electrode 12. In the detection part 13, the RNA aptamers 130 of the detection part 13 bind to hemoglobin in the analyte liquid so that the weight of the detection part 13 varies by an amount corresponding to the binding. This causes a change in the characteristics such as the phase of the SAW passing under the detection part 13. When the SAW whose characteristics have varied as described here reaches the second IDT electrode, a voltage corresponding to this is generated on the second IDT electrode. This voltage is outputted through the second extraction electrode 20, the wiring 7, and the like to the outside and then read by the external measuring instrument so that the property or the component of the analyte liquid can be investigated.

Here, in the sensor 100, guiding of the analyte liquid to the detection part 13 is achieved by a capillary phenomenon.

Specifically, as described above, when the second cover member 2 is joined to the intermediate cover member 1A, the passage 15 is formed in a long narrow tubular shape arranged in the lower face of the second cover member 2. Thus, with taking into consideration the kind of the analyte liquid, the construction materials of the intermediate cover member 1A and the second cover member 2, and the like, when the width, the diameter and the like of the passage 15 are set to be predetermined values, a capillary phenomenon can be generated in the long narrow tubular passage 15. For example, the width of the passage 15 is 0.5 mm to 3 mm and, for example, the depth thereof is 0.1 mm to 0.5 mm. Here, the passage 15 includes a downstream part (an extension part) 15b serving as a portion extending beyond the detection part 13. Further, the air release hole 18 connected to the extension part 15b is formed in the second cover member 2 Then, when the analyte liquid enters the passage 15, the air present in the passage 15 is released through the air release hole 18 to the outside.

When such a pipe where a capillary phenomenon is generated is formed by the cover member including the intermediate cover member 1A and the second cover member 2, at the time that the analyte liquid is brought into contact with the inflow part 14, the analyte liquid flows through the passage 15 and is then suctioned into the inside of the cover member. As such, the sensor 100 itself includes a suction mechanism for the analyte liquid, and hence suction of the analyte liquid can be achieved without the use of an instrument such as a pipette.

### (Lyophilic property of passage 15)

In the sensor 100 according to the present embodiment, the entirety of an inner surface of the passage 15 or a part of the inner surface, for example, a bottom surface, a wall surface, and the like of the passage 15, have a lyophilic property. When the inner surface of the passage 15 has a lyophilic property, a capillary phenomenon is easily generated and hence the analyte liquid is easily suctioned through the inflow part 14.

For example, it is preferable that the portion having a lyophilic property within the inner surface of the passage 15 may be constructed so that the contact angle with water may become 60° or smaller. When the contact angle is 60° or smaller, a capillary phenomenon is more easily generated and hence suction of the analyte liquid into the passage 15 becomes more reliable at the time that the analyte liquid is brought into contact with the inflow part. Detailed description is given below with reference to FIG. 3(a). FIG. 3(a) is an enlarged sectional view showing a part of the sensor 100 of FIG. 2 (b) .

For example, employable methods for imparting a lyophilic property to the inner surface of the passage 15 include: a method of performing lyophilic-property-imparting processing (hydrophilization) on the inner surface of the passage 15; a method of gluing a lyophilic film onto the inner surface of the passage 15; and a method of forming the cover member 2 constituting the passage 15 of a lyophilic material. Among these, when the method of performing lyophilic-property-imparting processing on the inner surface of the passage 15 and the method of gluing a lyophilic film onto the inner surface of the passage 15 are employed, the analyte liquid flows through the inside of the passage 15 along the lyophilic portion and hence a situation can be suppressed that the analyte liquid flows to an unintended portion. This permits high-precision measurement. Further, according to these methods, even when a cover member produced from a lyophobic (hydrophobic) material is employed, a capillary phenomenon can be generated. Thus, an advantage of wide variety of options for the material employed in the cover member is also obtained.

As the method of performing lyophilic-property-imparting processing on the inner surface of the passage 15, for example, in a case of hydrophilization processing, the functional groups on the surface may be changed by ashing of the inner surface of the passage 15 employing oxygen plasma, then a silane coupling agent may be applied, and then polyethylene glycol may finally be applied. As another method, surface processing employing a processing agent having phosphorylcholine may be performed on the inner surface of the passage 15.

Further, in the method of gluing a lyophilic film, a commercial polyester-based or polyethylene-based film having undergone hydrophilization processing may be employed as the lyophilic film. The lyophilic film may be formed only on an upper face, a side face, or a lower face of the passage 15 or, alternatively, a combination of these may be employed.

### (Positional relation between passage 15 and sensing element 3)

In the present embodiment, the passage 15 for analyte liquid has a depth of approximately 0.3 mm while the sensing element 3 has a thickness of approximately 0.3 mm. Thus, as shown in FIG. 2(b), the depth of the passage 15 and the thickness of the sensing element 3 are almost equal to each other. Thus, in a case where the sensing element 3 is directly placed in the passage 15, the passage 15 will be blocked. Accordingly, in the sensor 100, as shown in FIGS. 2(b) and 3, there is provided the element accommodation recess 5 which is defined by the first cover member 1 on which the sensing element 3 is mounted and the intermediate cover member 1A joined onto the first cover member 1. When the sensing element 3 is accommodated in the element accommodation recess 5, the passage 15 for analyte liquid is prevented from being blocked. That is, the depth of the element accommodation recess 5 is set to be comparable with the thickness of the sensing element 3 and then the sensing element 3 is mounted in the element accommodation recess 5, so that the passage 15 can be ensured.

From the perspective of sufficiently ensuring the passage 15 for analyte liquid, as shown in FIGS. 2(b) and 3, it is preferable that the height from the bottom face of the element accommodation recess 5 to the upper face of the substrate 10 is set equal to or smaller (lower) than the depth of the element accommodation recess 5. For example, in a case where the height of the upper face of the substrate 10 measured from the bottom face of the element accommodation recess 5 is set equal to the depth of the element accommodation recess 5, the bottom face of the passage 15 can be located substantially at the same height as the detection part 13 when the inside of the passage 15 is viewed from the inflow part 14.

Here, for example, the planar shape of the element accommodation recess 5 may be similar to the planar shape of the substrate 10. Then, it is preferable that the element accommodation recess 5 is formed somewhat larger than the substrate 10. More specifically, the element accommodation recess 5 has such a size that when the substrate 10 is mounted in the element accommodation recess 5, a gap of approximately 200 µm may be formed between a side face of the substrate 10 and the inner wall of the element accommodation recess 5.

For example, the sensing element 3 is fixed to the bottom face of the element accommodation recess 5 with a die bonding material composed mainly of epoxy resin, polyimide resin, silicone resin, or the like.

The end part 19e of the first extraction electrode 19 and the wiring 7 are electrically connected to each other through a metal thin wire 27 composed of Au or the like. Similar connection is performed also between the end part 20e of the second extraction electrode 20 and the wiring 7. Here, the connection between the first extraction electrode 19 or the second extraction electrode 20 and the wiring 7 is not limited to the method employing the metal thin wire 27. For example, an electrically conductive bonding material such as an Ag paste may be employed. A gap is provided in the connection part between the first extraction electrode 19 or the second extraction electrode 20 and the wiring 7. Thus, when the second cover member 2 is glued to the first cover member 1, breakage of the metal thin wire 27 is suppressed. The first extraction electrode 19, the second extraction electrode 20, the metal thin wire 27, and the wiring 7 are covered by an insulating member 28. Since the first extraction electrode 19, the second extraction electrode 20, the metal thin wire 27, and the wiring 7 are covered by the insulating member 28, corrosion of these electrodes and the like can be suppressed.

As described above, according to the sensor 100 of the present embodiment, since the sensing element 3 is accommodated in the element accommodation recess 5 of the first cover member 1, the passage 15 for analyte liquid extending from the inflow part 14 to the detection part 13 can be ensured so that the analyte liquid suctioned through the inflow part 13 by a capillary phenomenon or the like can be led to the detection part. That is, even when the sensing element 3 having a non-negligible thickness is employed, it is possible to provide the sensor 100 in which a suction mechanism is provided in itself.

Next, modified examples of the sensor 100 according to the embodiment are described below.

### <Modified examples>

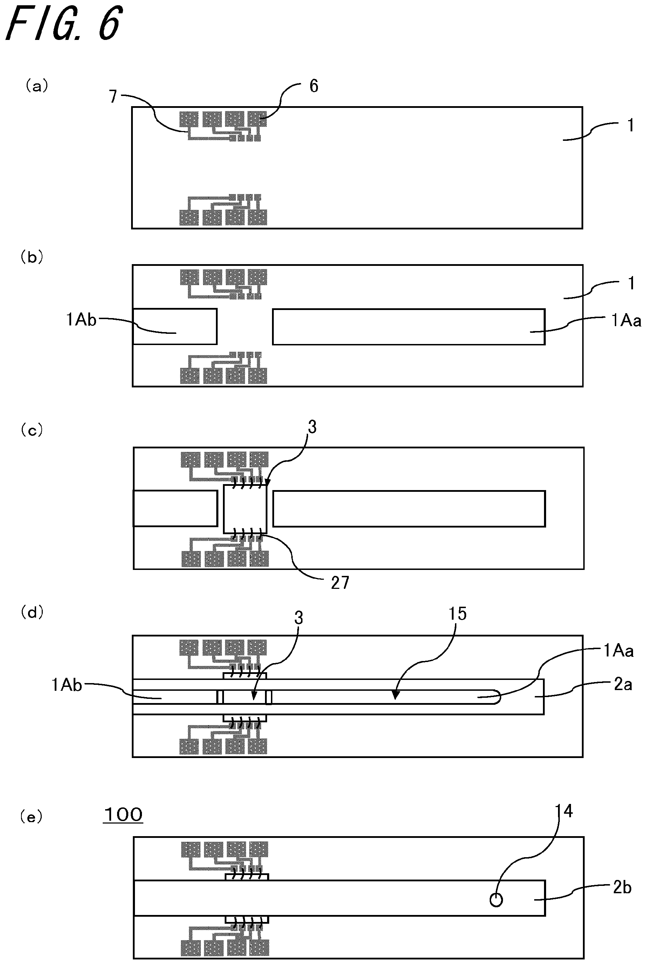

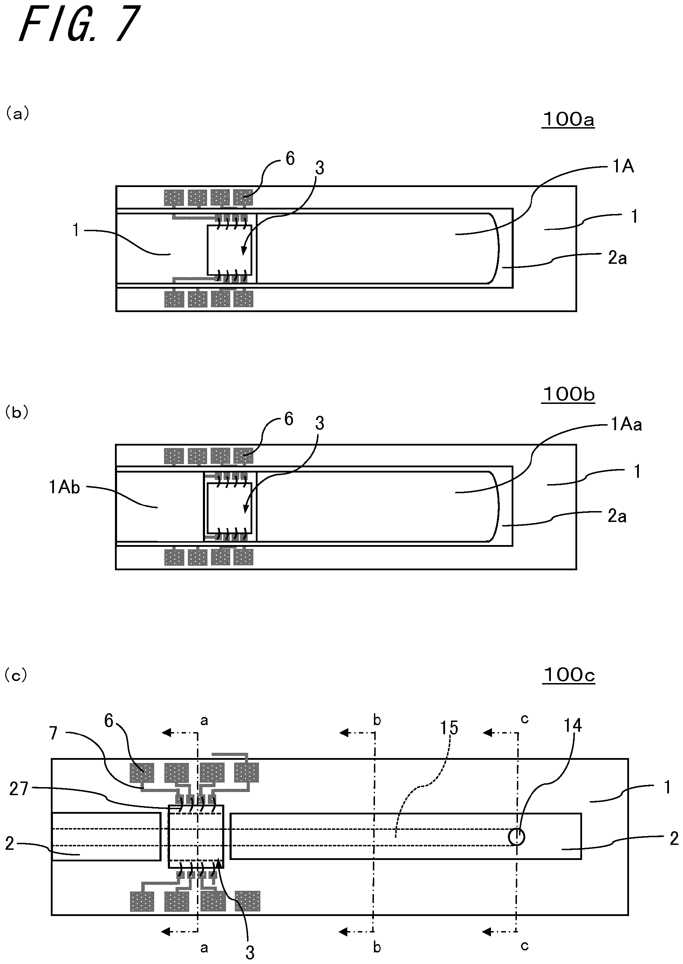

FIG. 7 shows plan views of sensors 100a, 100b, and 100c according to modified examples of the sensor 100 of FIG. 2. FIGS. 7(a) and 7(b) are views corresponding to FIG. 6(d), and FIG. 7(c) is a view corresponding to FIG. 2(a).

In contrast to the sensor 100 according to the above-mentioned embodiment, in the sensors 100a and 100b according to the present modified examples, the width of the intermediate cover member 1A and the width of the second cover member 2 is greater than the width of the sensing element 3. In the sensor 100a, as shown in FIG. 7(a), the intermediate cover member 1A (a second downstream part 1Ab) is not provided on the first cover member 1 in the downstream of the sensing element 3. In contrast, in the sensor 100b, as shown in FIG. 7(b), the intermediate cover member 1A (a second downstream part 1Ab) is provided on the first cover member 1 in the downstream of the sensing element 3.

Next, in the sensor 100c according to the present modified example, arrangement of the terminals 6 relative to the sensing element 3 is different from that in the sensor 100 according to the above-mentioned embodiment.

Specifically, in the sensor 100, as shown in FIG. 2, the terminals 6 are disposed on the air release hole 18 side of the sensing element 3 relative to the inflow part 14 side end. In contrast, in the sensor 100c of the present modified example, as shown in FIG. 7(c), at least a part of the terminals 6 is disposed on the inflow part 14 side of the sensing element 3 relative to the inflow part 14 side end.

Further, in the four terminals 6 aligned on the one side of the sensing element 3 with reference to the longitudinal direction of the passage 15, the lengths of the wirings 7 connected to the two outer side terminals 6 are substantially the same as each other and the lengths of the wirings 7 connected to the two inner side terminals 6 are substantially the same as each other. By virtue of this, it is possible to suppress variations in the signals obtained by the sensing element 3 depending on the lengths of the wirings 7. Further, for example, when a configuration is employed that one of the wirings 7 having substantially the same lengths is connected to the site of detecting the detection target in the detection part 13 of the sensing element 3 and the other one of the wirings 7 having substantially the same lengths is connected to the reference electrode for the detection target in the detection part of the sensing element 3, the above-mentioned variations in the signals can be suppressed and hence the reliability of detection can be improved.

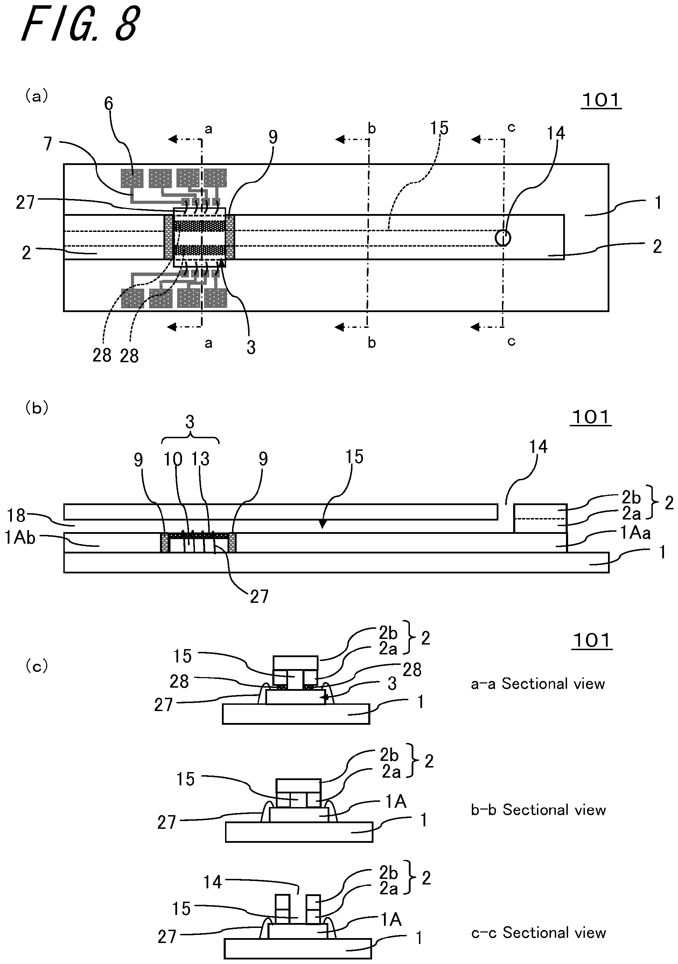

FIG. 8 is a diagram showing a sensor 101 according to a modified example of the sensor 100 of FIG. 2, wherein FIG. 8(a) is a plan view, FIG. 8(b) is a sectional view in the length direction, and FIG. 8(c) is a sectional view in the width direction.

In contrast to the sensor 100 according to the above-mentioned embodiment, in the sensor 101 according to the present modified example, the first IDT electrode 11 and the second IDT electrode 12 are covered by the insulating member 28.

The insulating member 28 contributes to oxidation prevention and the like of the first IDT electrode 11 and the second IDT electrode 12. For example, the insulating member 28 is formed of silicon oxide, aluminum oxide, zinc oxide, titanium oxide, silicon nitride, or silicon. For example, the thickness of the insulating member 28 is about 1/10 (10 to 30 nm) of the thickness of the first IDT electrode 11 and the second IDT electrode. The insulating member 28 may be formed over the entirety of the upper face of the substrate 10 in a state where the end part 19e of the first extraction electrode 19 and the end part 20e of the second extraction electrode 20 are exposed.

Further, in contrast to the sensor 100 according to the above-mentioned embodiment, in the sensor 101 according to the present modified example, a filling member 9 is provided in the gap between the sensing element 3 and the intermediate cover member 1A.

The filling member 9 may contain a material different from the intermediate cover member 1A and the substrate 10. For example, a resin material such as PDMS may be employed. Here, the filling member 9 does not have to be provided in an entire region of the gap between the sensing element 3 and the intermediate cover member 1A. For example, the filling member 9 may be provided only in a site corresponding to the passage 15. Since the filling member is located in the gap between the sensing element and the intermediate cover member, it is possible to suppress blocking of a capillary phenomenon caused by the gap so that the analyte liquid can more smoothly be suctioned toward the sensing element.

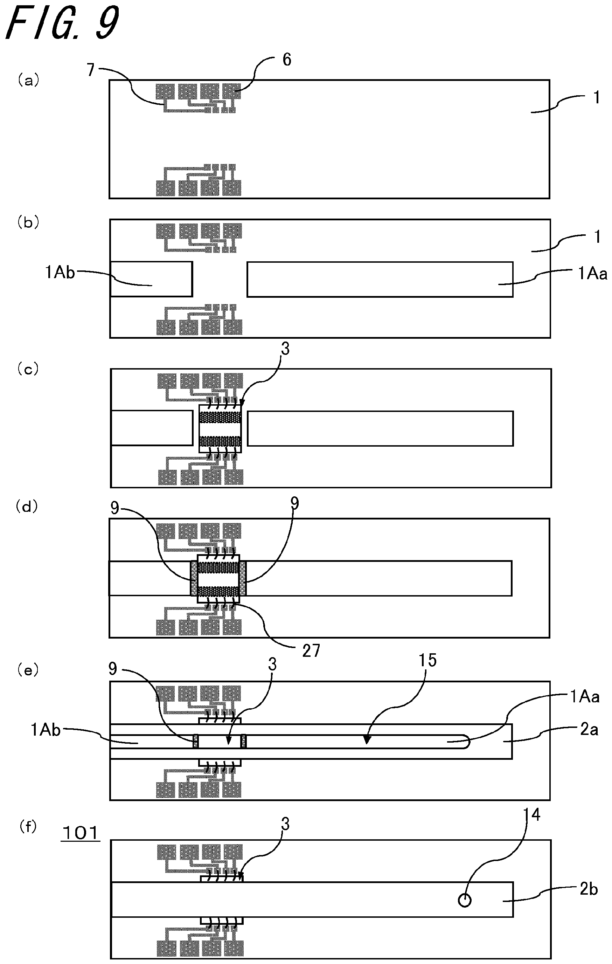

FIG. 9 is a plan view showing a production process of the sensor 101 of FIG. 8.

First, as shown in FIG. 9(a), the first cover member 1 on which the terminals 6 and the wirings 7 have been formed is prepared.

Then, as shown in FIG. 9(b), the intermediate cover member 1A is stacked onto the first cover member 1. Here, the intermediate cover member 1A is constructed from the first upstream part 1Aa and the first downstream part 1Ab.

Then, as shown in FIG. 9(c), the sensing element 3 is mounted between the first upstream part 1Aa and the first downstream part 1Ab of the intermediate cover member 1A by using the metal thin wires 27. Here, as for the steps of placing the intermediate cover member 1A and the sensing element 3 onto the first cover member 1, whichever step may be executed first.

Then, as shown in FIG. 9(d), the filling member 9 is disposed in the gap between the sensing element 3 and the intermediate cover member 1A.

Then, as shown in FIG. 9(e), the third substrate 2a of the second cover member 2 is stacked onto the intermediate cover member 1A.

Then, as shown in FIG. 9(a), the fourth substrate 2b is stacked onto the third substrate 2a so that the sensor 101 according to the present embodiment is produced.

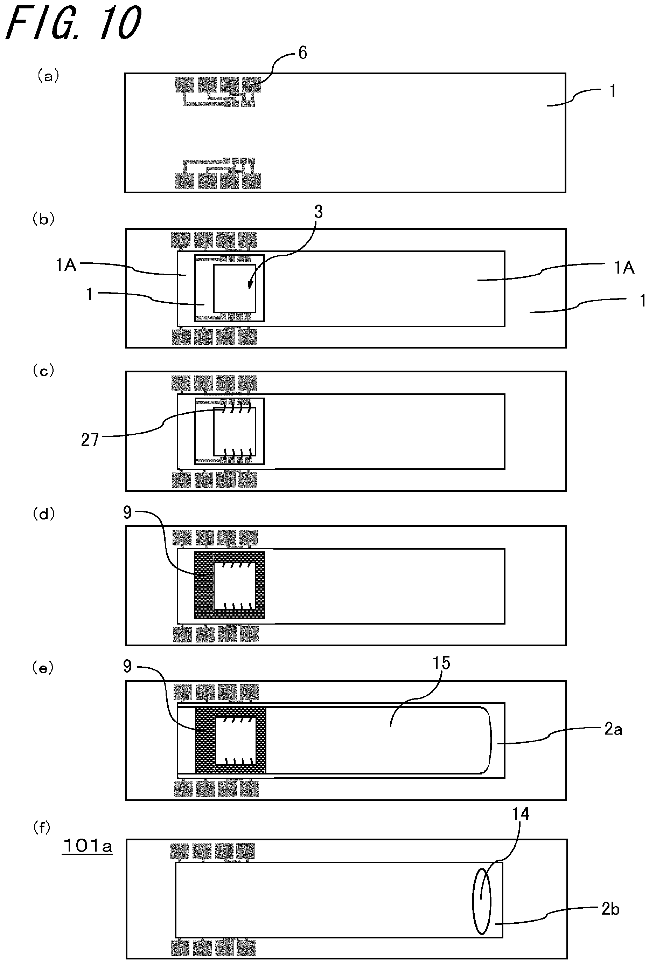

FIG. 10 is a diagram showing a sensor 101a according to a modified example of the sensor 100 of FIG. 2, in which a production process thereof is shown in particular.

In contrast to the sensor 100 according to the above-mentioned embodiment, in the sensor 101a according to the present modified example, the entire circumference of the sensing element 3 in a top view is surrounded by the intermediate cover member 1A. Then, as shown in FIGS. 10(d) and 10(e), the filling member 9 is located in the gap between the sensing element 3 and the intermediate cover member 1A so as to surround the outer periphery of the sensing element 3. By virtue of this, the level difference or the gap between the sensing element 3 and its surroundings in the passage 15 can be reduced so that the analyte liquid can smoothly flow on the sensing element 3. Further, the filling member 9 can cover a part of the wirings 7 and the lead wires 27 for connecting the sensing element 3 to the wirings 7, in a region of the sensing element 3 and the terminals 6. Thus, it is possible to suppress a decrease in the detection sensitivity caused by contact of these members with the analyte liquid.

Here, in the present modified example, the intermediate cover member 1A and the sensing element 3 are formed as shown in FIG. 10(b) and, after that, the sensing element 3 and the wirings 7 are connected to each other through the lead wires 27 as shown in FIG. 10(c). Alternatively, the sensing element 3 may be formed, then the sensing element 3 and the wirings 7 may be connected to each other through the lead wires 27 and, after that, the intermediate cover member 1A may be formed.

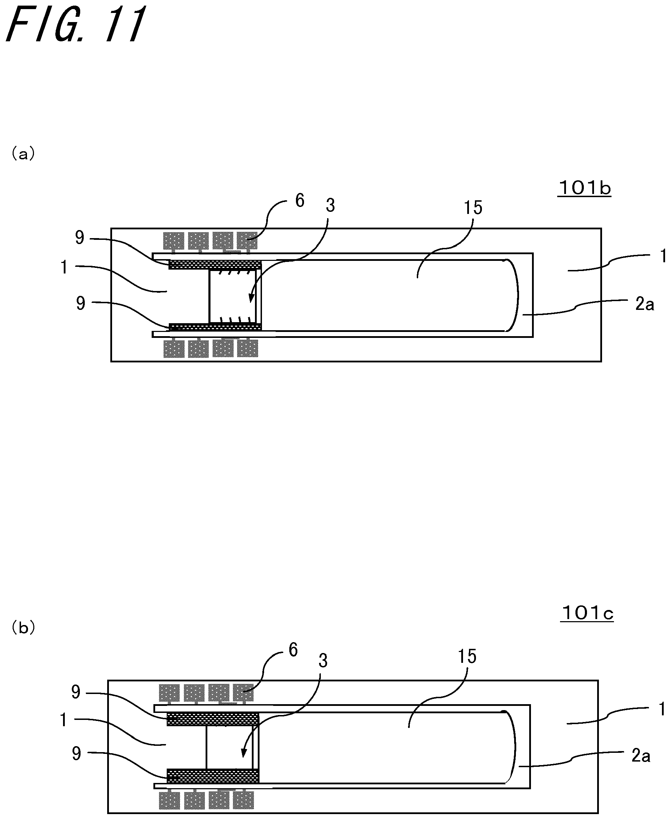

FIG. 11 shows plan views of sensors 101b and 101c according to modified examples of the sensor 100 of FIG. 2, which correspond to FIG. 6(e).

In contrast to the sensor 100 according to the above-mentioned embodiment, in the sensors 101b and 101c according to the present modified example, as shown in FIGS. 11(a) and 11(b), the filling member 9 is located along the longitudinal direction of the passage 15 within the gap between the sensing element 3 and the intermediate cover member 1A. By virtue of this, the level difference between the sensing element 3 and both sides thereof can be made low or the gap can be made narrow. Thus, the analyte liquid can smoothly flow to the sensing element 3 from the sides too. Further, the filling member 9 can cover a part of the wirings 7 and the lead wires 27 for connecting the sensing element 3 to the wirings 7, in a region of the sensing element 3 and the terminals 6. Thus, it is possible to suppress a decrease in the detection sensitivity caused by contact of these members with the analyte liquid.

Further, as shown in FIG. 11(b), the filling member 9 may cover not only the gap between the sensing element 3 and the intermediate cover member 1A but also a portion of the lead wires 27 for connecting the sensing element 3 to the wirings 7 which portion is located on an upper face of the sensing element 3 (the substrate 10). By virtue of this, it is possible to further suppress a decrease in the detection sensitivity could be caused by contact of the lead wires 27 with the analyte liquid.

### EXAMPLES

Examples of the RNA aptamer according to the above-mentioned embodiment and the sensor employing the same are described below.

### First example

Evaluation was performed by using Biacore T200 (manufactured by GE Healthcare) serving as an equipment for an intermolecular interaction analysis. This equipment includes four flow cells.

As a chip (a substrate) for evaluation, a Series S Sensor Chip SA (manufactured by GE Healthcare) was employed.

A procedure of immobilizing various kinds of RNA aptamers onto the Series S Sensor Chip SA and then detecting hemoglobin is described below.

Here, Human Hemoglobin full length protein (ab77858) manufactured by Abcam plc. was employed as a hemoglobin sample.

Further, a solution (referred to as HBS-P(+MgCl2), hereinafter) obtained by adding MgCl2 to HBS-P (manufactured by GE Healthcare) so that the MgCl2 content may become 1 mM was employed as a running buffer solution.
(1) All flow cells were rinsed with 10 mM NaOH at a flow rate of 20 µl/min for 1 minute.
(2) The above-mentioned procedure (1) was repeated twice.
(3) 5-µM 5'-TTTTTTTTTTTTTTTTTT whose 5'-terminal had a biotin was supplied to one (referred to as Fc2, hereinafter) of the flow cells at a flow rate of 5 µl/min for 6 minutes so that immobilization was performed. At that time, dilution was performed by using an HBS-P(+MgCl2) solution.
(4) 5-µM 5'-TTTTTTTTTTTTTTTTTT whose 5'-terminal had a biotin was supplied to Fc2 at a flow rate of 5 µl/min for 2 minutes so that immobilization was performed.
(5) 1 mg/ml biotin was supplied to one (referred to as Fc1, hereinafter) of the flow cells at a flow rate of 5 µl/min for 6 minutes so that immobilization was performed. At that time, dilution was performed by using an HBS-P(+MgCl2) solution.
(6) All flow cells were rinsed with 10-mM NaOH at a flow rate of 20 µl/min for 1 minute.
(7) The above-mentioned procedure (6) was repeated once.
(8) 2-µM RNA aptamer was supplied to Fc2 at a flow rate of 5 µl/min for 4 minutes so that immobilization was performed. Here, the employed RNA aptamer was an RNA aptamer in which AAAAAAAAAAAAAAAAAA was added to the 3'-terminal, the 2' positions of C and U were replaced with a fluorine group, and annealing was performed in advance. At that time, dilution was performed by using an HBS-P(+MgCl2) solution.
(9) A hemoglobin sample having been diluted into 1 µM with HBS-P(+MgCl2) was supplied to all flow cells at a flow rate of 20 µl/min for 2 minutes. After that, the dissociation state thereof was monitored for 1 minute.
(10) A hemoglobin sample having been diluted into 10 µM with HBS-P(+MgCl2) was supplied to all flow cells at a flow rate of 20 µl/min for 2 minutes. After that, the dissociation state thereof was monitored for 1 minute.
(11) All flow cells were rinsed with 10-mM NaOH at a flow rate of 20 µl/min for 1 minute. As a result of this work, the RNA aptamers were detached and hence the Series S Sensor Chip SA was returned to the state where the above-mentioned procedure (7) was completed.
(12) After that, the above-mentioned procedures (8) to (11) were repeated so that various kinds of RNA aptamers listed in Table 1 were sequentially evaluated. These evaluation results are listed in Table 2.

**[Table 1]**

| Sample No. | Name | Sequence |
|---|---|---|
| No.1 | 6R_0 | GGGAUAGGAUCCACAUCUACGUAUUACGGCAUUAUUAGGACCAAUACGUGGUUGACCCUCCCUCAACGACGCAG UUCACUGCAGACUUGACGAAGCUU |
| No.2 | 6R_2 | GGGAUAGGAUCCACAUCUACGUAUUAGGACCAUGUAGAGUAGAUCGCCAUCCCAGCAGGUUGCAGUUUCAUGUU CACUGCAGACUUGACGAAGCUU |
| No.3 | 6R_3 | GGGAUAGGAUCCACAUCUACGUAUUAUUAGGACCAAUGCGUAGCGCGACCUUUCCCGAAAGGACUGUUCAUGUU CACUGCAGACUUGACGAAGCUU |
| No.4 | 6R_4 | GGGAUAGGAUCCACAUCUACGUAUUAGGACCACGUAGGUAUACGGCGUACUCCCCGGGUCUGCAAAGUUUUUCA CUGCAGACUUGACGAAGCUU |
| No.5 | 6R_5 | GGGAUAGGAUCCACAUCUACGUAUUAGGACCACGCGGGUGUCGAUUCGUUAUCCCCAGGAUUGCAGUACAUUCA CUGCAGACUUGACGAAGCUU |
| No.6 | 6R_6 | GGGAUAGGAUCCACAUCUACGUAUUAGGACCAUGUAGAUGUCGCUAACUCCCCUCGUCUGCAAACUUUCCUUCA CUGCAGACUUGACGAAGCUU |
| No.7 | 6R_7 | GGGAUAGGAUCCACAUCUACGUAUUAGGACCACGUAGUUGGGUCACAUUCCCCGCCAAGCCAGAGAUUAAUUCA CUGCAGACUUGACGAAGCUU |
| No.8 | 6R_8 | GGGAUAGGAUCCACAUCUACGUAUUAGGACCACGCGGAGUGGAUUCACCGGAUCCCGCAUGUCUGUUUACUUCA CUGCAGACUUGACGAAGCUU |
| No.9 | 6R_9 | GGGAUAGGAUCCACAUCUACGUAUUAGGACCAUGUAGGUGGACACAUCCCAACGCAAGCGCGACUAUUAAUUCA CUGCAGACUUGACGAAGCUU |
| No.10 | 6R_10 | GGGAUAGGAUCCACAUCUACGUAUUAUUAUUAGGACAAAUACGGGGUGUGCCCGCCGCUAAGACUGCGCUUCAC UGCAGACUUGACGAAGCUU |
| No.11 | 6R_11 | GGGAUAGGAUCCACAUCUACGUAUUAGGAGUAUCCCCCUGCAGAGUUGUCUAUUAGGACCAGACAUUUUUCACU GCAGACUUGACGAAGCUU |
| No.12 | 6R_12 | GGGAUAGGAUCCACAUCUACGUAUUAGGACCAUGUAGACGGAUCACUCGCCCACGCAAGGCUGCAAUUUCACU GCAGACUUGACGAAGCUU |
| No.13 | 6R_13 | GGGAUAGGAUCCACAUCUACGUAUUAGGACCACGUAGAAAUCUCAGUCCCCUAGUCAGCGGCUAACUUUUCACU GCAGACUUGACGAAGCUU |
| No.14 | 6R_14 | GGGAUAGGAUCCACAUCUACGUAUUAGGACCACGUAGGUGCACCACUAAUCCCUCAGGUCUGCAACUUUUCACU GCAGACUUGACGAAGCUU |
| No.15 | 6R_15 | GGGAUAGGAUCCACAUCUACGUAUUAUAGGACCACGUAGCAGACCCCAAUCCCUGUCAGUCACAGUUAUUCACU GCAGACUUGACGAAGCUU |
| No.16 | 6R_16 | GGGAUAGGAUCCACAUCUACGUAUUAGGACCACGUAGAACCCCUAGUCCCGCUACGGUUCAAUCUGUUUCACU GCAGACUUGACGAAGCUU |
| No.17 | 6R_17 | GGGAUAGGAUCCACAUCUACGUAUUAGGACCACGUAGUUAAAUCCCACCCCGACCCGCUGUUUGCGUUUUCACU GCAGACUUGACGAAGCUU |
| No.18 | 6R_18 | GGGAUAGGAUCCACAUCUACGUAUUAGGACCAUGUAGAGCGUCCACAUCCCCAGGUCUGCUGGUCGUUUCACU GCAGACUUGACGAAGCUU |
| No.19 | 6R_19 | GGGAUAGGAUCCACAUCUACGUAUUAGGACCACGUGGAUGGCGCGAAUUCCCCUUGAUCUGCAGGUGUUUCACU GCAGACUUGACGAAGCUU |
| No.20 | 6R_20 | GGGAUAGGAUCCACAUCUACGUAUUAGGACCACGUAGUACACUCCCGUCCCCGACAAACGCAGUAUUUUCACU GCAGACUUGACGAAGCUU |
| No.21 | 6R_21 | GGGAUAGGAUCCACAUCUACGUAUUAUAGGACCACGUAGAGAACUUUCCCGAGCGACAAGGGCCUGCAUUCACU GCAGACUUGACGAAGCUU |
| No.22 | 6R_22 | GGGAUAGGAUCCACAUCUACGUAUUAGGACCACGUAGAGCUCUCCCCGCCAAAGUCGCAUGAACGGUUUCACU GCAGACUUGACGAAGCUU |
| No.23 | 6R_23 | GGGAUAGGAUCCACAUCUACGUAUUAGGACCACGUAGUCCAAUCCUCCAUUCCCAAAAGCGCAGAUUUUCACUG CAGACUUGACGAAGCUU |
| No.24 | 6R_24 | GGGAUAGGAUCCACAUCUACGUAUUAGGACCACGUAGACAAUCCUAUCGCACGAAAGUCGCACAUCUUUUCACUG CAGACUUGACGAAGCUU |
| No.25 | 6R_25 | GGGAUAGGAUCCACAUCUACGUAUUAGGACCAUGCAGAUGCUUGGUUCAUAUCCCUAAGUCACCAGUUUCACU GCAGACUUGACGAAGCUU |
| No.26 | 6R_26 | GGGAUAGGAUCCACAUCUACGUAUUAGGACCACGUAGACGGCAACGCUAUCCGCGCAAAGAGCAGUUUUCACU GCAGACUUGACGAAGCUU |
| No.27 | 6R_27 | GGGAUAGGAUCCACAUCUACGUAUUAGGACCACGUAGACCACUGCGGUCCAAAUCCCCCUCUCAAGUUUCACU GCAGACUUGACGAAGCUU |
| No.28 | 6R_28 | GGGAUAGGAUCCACAUCUACGUAUUACGGCAUUAUUAGGACCAAUGCUGAGUACGGCGCACAGCUUUUUCACU GCAGACUUGACGAAGCUU |
| No.29 | 6R_29 | GGGAUAGGAUCCACAUCUACGUAUUAUAGGACCAUGUAGGAGUCGGACCCACGCCCCGAGCUGCAGUCUUCACU GCAGACUUGACGAAGCUU |
| No.30 | 6R_30 | GGGAUAGGAUCCACAUCUACGUAUUAGGACCACGUAGGUGGCAAUGAUCCCACAGCGUGUAGAGUUUUUCACU GCAGACUUGACGAAGCUU |
| No.31 | 6R_31 | GGGAUAGGAUCCACAUCUACGUAUUAGGACCACGUAGAUUCCUCUAUCCUGUUCGAGUCUCCAGACUUUUCACU GCAGACUUGACGAAGCUU |
| No.32 | 6R_32 | GGGAUAGGAUCCACAUCUACGUAUUAGGACCACGUAGAAGGGCACGGUUUGCUAUCCCCCAUCAAGUUUCACU GCAGACUUGACGAAGCUU |
| No.33 | 6R_33 | GGGAUAGGAUCCACAUCUACGUAUUAGGACCACGCUAGAUGGACCCGUACGCCCCAAGUCGACAGGUUUUCACU GCAGACUUGACGAAGCUU |
| No.34 | 6R_34 | GGGAUAGGAUCCACAUCUACGUAUUAGGACCACGUAGGCCCGUUGGCAUCCCCGAAAGUCGAACAGUUUCACU GCAGACUUGACGAAGCUU |
| No.35 | 6R_35 | GGGAUAGGAUCCACAUCUACGUAUUAUAUUAGGACAAAUCCGUGGUGUGCCCGCCGCUAAGACUGCGCUUCACU GCAGACUUGACGAAGCUU |
| No.36 | 6R_36 | GGGAUAGGAUCCACAUCUACGUAUUAUAGGACCACGUAGGUGGACUCCCAACCAACGAACAUAGCCUGUUCACU GCAGACUUGACGAAGCUU |
| No.37 | 6R_37 | GGGAUAGGAUCCACAUCUACGUAUUAGGACCACGCAGAUGGUUCUCACCCGAAGUCAUGCGCUACAUUUCACU GCAGACUUGACGAAGCUU |
| No.38 | 6R_38 | GGGAUAGGAUCCACAUCUACGUAUUAGGACCACGUAGAACCCGCCCCAAAGUCUACAGCAUGUAGGUUUUCACU GCAGACUUGACGAAGCUU |
| No.39 | 6R_39 | GGGAUAGGAUCCACAUCUACGUAUUAGGACCACGUAGUAGAGAUCCCGCACCGCAGUCUGUAGGCUUUUUCACU GCAGACUUGACGAAGCUU |
| No.40 | 6R_40 | GGGAUAGGAUCCACAUCUACGUAUUAUAGGACCACGUAGUGUCGGACCUUACCCCUCAGGUCGGGUACUUCACU GCAGACUUGACGAAGCUU |
| No.41 | 6R_41 | GGGAUAGGAUCCACAUCUACGUAUUAGGACCACGUAGACCUACCCCCUUCAUGUCCAGUGCAGACAUUUUCACU GCAGACUUGACGAAGCUU |
| No.42 | 6R_42 | GGGAUAGGAUCCACAUCUACGUAUUAGGACCAUGUAGAUUAGCGCAACCUGUCCGCUCCAGUCAGGUAUUCACU GCAGACUUGACGAAGCUU |
| No.43 | 6R_43 | GGGAUAGGAUCCACAUCUACGUAUUAGGACCACGUAGGCCAAAUAUCCCGCUUACAGUCAGCACGUUUUCACU GCAGACUUGACGAAGCUU |
| No.44 | 6R_44 | GGGAUAGGAUCCACAUCUACGUAUUAGGACCACGUAGAUCGGCUCCACAGACGGUCAGAACUGCAAUUUCACU GCAGACUUGACGAAGCUU |
| No.45 | 6R_45 | GGGAUAGGAUCCACAUCUACGUAUUAGGACCACGUAGACAAGCCCUGUCCUAGUAACCUCAAGUUUCAUUCACU GCAGACUUGACGAAGCUU |
| No.46 | 6R_46 | GGGAUAGGAUCCACAUCUACGUAUUAUGUGCUAUUAGGACCAGCACAUACGGACGCCACAGUCUGCGAUUCACU GCAGACUUGACGAAGCUU |
| No.47 | 6R_47 | GGGAUAGGAUCCACAUCUACGUAUUAGGACCACGUAGUGUCUCGCUCCAAACCCGGCAAUCUCAAGUUUCACU GCAGACUUGACGAAGCUU |
| No.48 | 6R_48 | GGGAUAGGAUCCACAUCUACGUAUUAGGACCACGUAGGUGGACCCAGAUUCCGCAACCCGUAAAGUCCUUCACU GCAGACUUGACGAAGCUU |
| No.49 | 6R_49 | GGGAUAGGAUCCACAUCUACGUAUUAGGACCACGUAGAGACUACCCUCCCCCAAGUCGUAGAACGUUUUCACU GCAGACUUGACGAAGCUU |
| No.50 | 6R_50 | GGGAUAGGAUCCACAUCUACGUAUUAGGACCACGUAGACCUCCCUAGUUCAGCGAGCAGGAAGUUCUUCACUGC AGACUUGACGAAGCUU |

**[Table 2]**

| Sample No. | Name | Immobilization amount RU | Detected Hb amount RU | Number of bases | Hb binding ratio per one aptamer | Evaluation |
|---|---|---|---|---|---|---|
| No.1 | 6R_0 | 2274.70 | -34.41 | 116 | - | Bad |
| No.2 | 6R_2 | 2291.64 | 765.13 | 114 | 0.192 | Good |
| No.3 | 6R_3 | 2049.96 | 765.06 | 114 | 0.214 | Excellent |
| No.4 | 6R_4 | 2614.26 | 592.96 | 112 | 0.128 | Good |
| No.5 | 6R_5 | 2008.55 | 680.04 | 112 | 0.191 | Good |
| No.6 | 6R_6 | 2576.28 | 281.15 | 112 | 0.062 | Not bad |
| No.7 | 6R_7 | 1784.58 | 572.61 | 112 | 0.181 | Good |
| No.8 | 6R_8 | 2299.43 | 828.00 | 112 | 0.203 | Excellent |
| No.9 | 6R_9 | 1962.46 | 870.35 | 112 | 0.250 | Excellent |
| No.10 | 6R_10 | 2245.76 | -72.51 | 111 | - | Bad |
| No.11 | 6R_11 | 2165.05 | 835.88 | 110 | 0.214 | Excellent |
| No.12 | 6R_12 | 2215.42 | 824.15 | 110 | 0.206 | Excellent |
| No.13 | 6R_13 | 2294.53 | 619.19 | 110 | 0.150 | Good |
| No.14 | 6R_14 | 2483.66 | 658.35 | 110 | 0.147 | Good |
| No.15 | 6R_15 | 1924.02 | -92.84 | 110 | - | Bad |
| No.16 | 6R_16 | 2093.08 | 428.00 | 110 | 0.113 | Good |
| No.17 | 6R_17 | 1976.60 | 635.27 | 110 | 0.178 | Good |
| No.18 | 6R_18 | 2442.80 | 684.49 | 110 | 0.155 | Good |
| No.19 | 6R_19 | 2355.82 | 601.32 | 110 | 0.141 | Good |
| No.20 | 6R_20 | 2070.44 | 657.27 | 110 | 0.176 | Good |
| No.21 | 6R_21 | 1972.81 | -94.51 | 110 | - | Bad |
| No.22 | 6R_22 | 2622.28 | 857.24 | 110 | 0.181 | Good |
| No.23 | 6R_23 | 2032.11 | 611.81 | 110 | 0.167 | Good |
| No.24 | 6R_24 | 2185.90 | 669.67 | 110 | 0.170 | Good |
| No.25 | 6R_25 | 2130.00 | 634.67 | 110 | 0.165 | Good |
| No.26 | 6R_26 | 2269.33 | 529.16 | 110 | 0.129 | Good |
| No.27 | 6R_27 | 2032.41 | 601.93 | 110 | 0.164 | Good |
| No.28 | 6R_28 | 2717.64 | 1136.61 | 110 | 0.232 | Excellent |
| No.29 | 6R_29 | 2269.26 | -39.16 | 110 | - | Bad |
| No.30 | 6R_30 | 2523.18 | 767.74 | 110 | 0.169 | Good |
| No.31 | 6R_31 | 2004.88 | 504.46 | 110 | 0.139 | Good |
| No.32 | 6R_32 | 2138.49 | 649.63 | 110 | 0.168 | Good |
| No.33 | 6R_33 | 2153.75 | 876.03 | 110 | 0.225 | Excellent |
| No.34 | 6R_34 | 2346.63 | 565.43 | 110 | 0.134 | Good |
| No.35 | 6R_35 | 2221.07 | -61.97 | 110 | - | Bad |
| No.36 | 6R_36 | 2267.17 | -37.13 | 110 | - | Bad |
| No.37 | 6R_37 | 2139.49 | 577.36 | 110 | 0.150 | Good |
| No.38 | 6R_38 | 2454.71 | 658.28 | 110 | 0.149 | Good |
| No.39 | 6R_39 | 2079.71 | 701.19 | 110 | 0.187 | Good |
| No.40 | 6R_40 | 2017.40 | -44.39 | 110 | - | Bad |
| No.41 | 6R_41 | 1889.81 | 393.23 | 110 | 0.115 | Good |
| No.42 | 6R_42 | 2624.07 | 881.32 | 110 | 0.186 | Good |
| No.43 | 6R_43 | 2395.95 | 629.54 | 110 | 0.146 | Good |
| No.44 | 6R_44 | 2066.93 | 579.37 | 110 | 0.155 | Good |
| No.45 | 6R_45 | 1940.37 | 435.69 | 110 | 0.124 | Good |
| No.46 | 6R_46 | 2096.64 | 739.65 | 110 | 0.196 | Good |
| No.47 | 6R_47 | 1843.48 | 446.71 | 110 | 0.134 | Good |
| No.48 | 6R_48 | 2022.80 | 600.75 | 110 | 0.165 | Good |
| No.49 | 6R_49 | 2198.20 | 516.37 | 110 | 0.130 | Good |
| No.50 | 6R_50 | 2424.36 | 660.36 | 108 | 0.148 | Good |

Table 2 lists the amount of immobilization of the aptamer and the detected amount of Hb as well as the Hb binding ratio per one RNA aptamer. Then, "Excellent" indicates that the value of binding ratio is 0.2 or higher, "Good" indicates that the value is 0.1 or higher and lower than 0.2, "Not bad" indicates that the value is higher than 0 and lower than 0.1, and "Bad" indicates that the value is 0 or lower.

According to this, as shown in Table 1, it has been recognized that when RNA aptamers having a sequence of linkage order of UAUUAGGACCA are employed, these RNA aptamers bind to Hb. In particular, the RNA aptamers Nos. 3, 8, 9, 11, 12, 28, and 33 had a high binding strength to Hb.

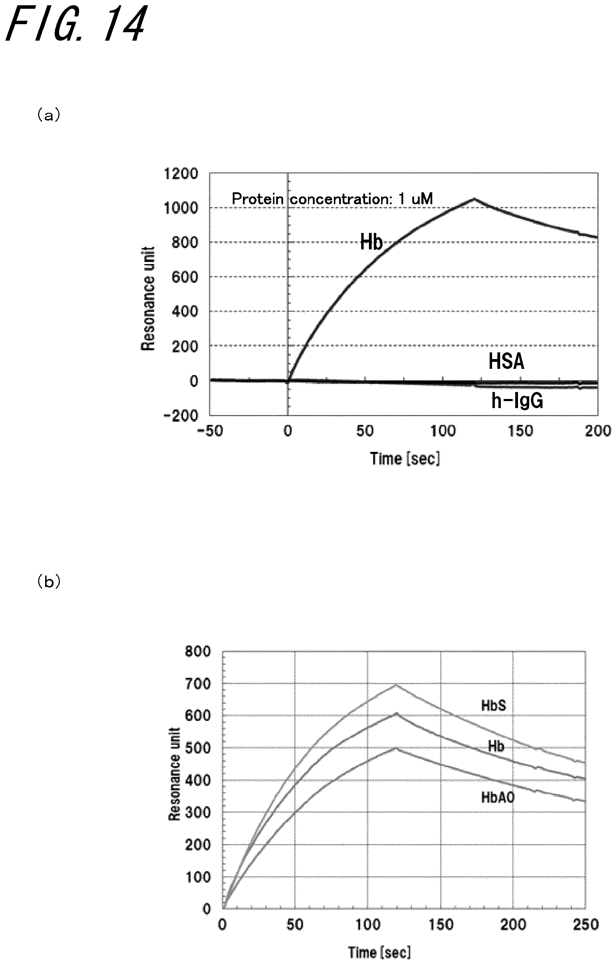

Further, FIG. 14(a) shows results of the cases where one of three kinds consisting of hemoglobin, human serum albumin (HSA), and human IgG (h-IgG) was employed as a sample in the above-mentioned procedure (9). According to this, it has been recognized that the RNA aptamer having a sequence of linkage order of UAUUAGGACCA specifically binds to hemoglobin as described above and has a tendency not to bind to human serum albumin (HSA) and human IgG (h-IgG).

FIG. 14(b) shows results of the cases that binding of the RNA aptamer 6R_11 listed in Table 1 to fractionation components of hemoglobin was measured in the above-mentioned procedure (9). Specifically, at time point 0 second, each of Hb, HbA0, and HbS was injected as a sample into each flow cell. Each data up to 120 seconds indicates binding between the aptamer and the target. Then, the data posterior to 120 seconds indicates a dissociation signal. The vertical axis indicates the amount of binding to the aptamer. According to this, it has been recognized that the RNA aptamer 6R_11 binds to at least HbA0 and HbS among the fractionation components of Hb.

### Second example

Evaluation was performed by using Biacore X (manufactured by GE Healthcare) serving as an equipment for an intermolecular interactionanalysis. This equipment includes two flow cells.

As a chip (a substrate) for evaluation, a Sensor Chip SA (manufactured by GE Healthcare) was employed.

A procedure of immobilizing an RNA aptamer onto the Series Chip SA and then detecting hemoglobin is described below.

Here, the hemoglobin sample and the running buffer solution employed here were the same as those in the first example.

Procedures (1) to (7) were basically the same as the procedures (1) to (7) of the first example mentioned above. However, in procedure (2), the above-mentioned (1) was repeated three times. Further, in procedure (7), the above-mentioned procedure (6) was repeated once.
(8) Similarly to the experiment described above, 2-µM RNA aptamer were supplied to Fc2 at a flow rate of 5 µl/min for 4 minutes so that immobilization was performed. At that time, dilution was performed by using an HBS-P(+MgCl2) solution.
(9) A hemoglobin sample having been diluted into 1 µM with HBS-P(+MgCl2) was supplied to the flow cells at a flow rate of 20 µl/min for 2 minutes. After that, the dissociation state thereof was monitored for 1 minute 30 seconds.
(10) All flow cells were rinsed with 10-mM NaOH at a flow rate of 20 µl/min for 1 minute. As a result of this work, the RNA aptamers were detached and hence the Series Chip SA was returned to the state where the procedure (7) was completed.
(11) After that, the above-mentioned procedures (8) to (10) were repeated so that various kinds of RNA aptamers listed in Table 3 were sequentially evaluated. These evaluation results are listed in Table 4.

**[Table 3]**

| Sample No. | Name | Sequence |
|---|---|---|
| No.101 | 6R_28_L | CGUACUCAGCAUUGGUCCUAAUAAUGCCGUAAUACGCCCUAUAGUGAGUCGUAUUAUC |
| No.102 | 6R_33_L1 | GGGCGUACGGGUCCAUCUAGCGUGGUCCUAAUACGUAGAUGUGGAUCCUAUCCCUAUAGUGAGUCGUAUU AUC |
| No.103 | 6_R_33_L2 | GGGUCCAUCUAGCGUGGUCCUAAUACGUAGAUGUGGAUCCCUAUAGUGAGUCGUAUUAUC |
| No.104 | 6R_3_L1 | GGGAAAGGUCGCGCUACGCAUUGGUCCUAAUAAUACGUAGAUGUGGAUCCUAUCCCUAUAGUGAGUCGUA UUAUC |
| No.105 | 6R_3_L2 | CUACGCAUUGGUCCUAAUAAUACGUAGCCCUAUAGUGAGUCGUAUUAUC |
| No.106 | 6R_11_L1 | CUGCAGUGAAAAAUGUCUGGUCCUAAUAGACAACUCUGCAGCCCUAUAGUGAGUCGUAUUAUC |

**[Table 4]**

| Sample No. | Name | Immobilization amount RU | Detected Hb amount RU | Number of bases | Hb binding ratio per one aptamer | Evaluation |
|---|---|---|---|---|---|---|
| No.101 | 6R_28_L | 2270 | 1380 | 57 | 0.175 | Good |
| No.102 | 6R_33_L1 | 1990 | 980 | 72 | 0.179 | Good |
| No.103 | 6R_33_L2 | 2100 | 1160 | 59 | 0.164 | Good |
| No.104 | 6R_3_L1 | 2210 | 1090 | 74 | 0.184 | Good |
| No.105 | 6R_3_L2 | 2380 | 930 | 48 | 0.095 | Not bad |
| No.106 | 6R_11_L1 | 2310 | 1360 | 62 | 0.184 | Good |

Similarly to Table 2 mentioned above, Table 4 lists the amount of immobilization of the aptamer and the detected amount of Hb as well as the Hb binding ratio per one RNA aptamer. Then, "Excellent" indicates that the value of binding ratio is 0.2 or higher, "Good" indicates that the value is 0.1 or higher and lower than 0.2, "Not bad" indicates that the value is higher than 0 and lower than 0.1, and "Bad" indicates that the value is 0 or lower.

According to this, as shown in Table 4, it has been recognized that when RNA aptamers having a sequence of linkage order of UAUUAGGACCA are employed, these RNA aptamers specifically bind to Hb. In particular, the RNA aptamers Nos. 101, 102, 103, 104, and 106 had a high binding strength to Hb. Further, it has been recognized that similarly to the various aptamers in the first example mentioned above, various short-chain aptamers in the present example also specifically bind to Hb.

### Third example (example that biotin was provided at terminal)

A procedure of immobilizing the RNA aptamer onto a mounting chip and then detecting hemoglobin is described below. Here, in the present example, an RNA aptamer having a sequence of a part of 6R_28 listed in Table 1 was employed. Specifically, an RNA aptamer composed of a sequence of CGUAUUACGGCAUUAUUAGGACCAAUGCUGAGUACG was employed. Here, the same RNA aptamer was employed also in a fourth example and a fifth example described later.
(1) The sensing element 3 was prepared. Au (gold) was employed as the immobilization membrane 13a.
(2) The region containing the detection part 13 was rinsed with a piranha solution (a mixture solution of concentrated sulfuric acid and 30% hydrogen peroxide).
(3) The region containing the detection part 13 was rinsed with water and ethanol.
   In the subsequent procedures (4) to (7) described below, the aptamer was immobilized onto the immobilization membrane Au.
(4) 3,3'-dithiodipropionic acid was dripped onto the region containing the detection part 13. Then, the chip was left still for 10 minutes and, after that, rinsed with water.
(5) A mixture liquid of a 1-ethyl-3(3-dimethylaminopropyl)carbodiimide aqueous solution and an N-hydroxysuccinimide aqueous solution was dripped onto the region containing the detection part 13. Then, the chip was left still for 20 minutes and, after that, rinsed with water so that active etherification was performed on the carboxyl group located at a terminal of 3,3'-dithiodipropionic acid.
(6) A solution obtained by dissolving streptavidin having a concentration of 0.1 mg/mL into a 1-mM HEPES buffer (pH 8.0) liquid was dripped onto the region containing the detection part 13. Then, the chip was left still for 20 minutes and, after that, rinsed with the 1-mM HEPES buffer (pH 8.0) liquid so that an amide linkage was formed between the etherified carboxyl group and the amino residue in the streptavidin.
(7) The 1-mM HEPES buffer (pH 8.0) liquid was substituted with HBS-P(+MgCl2). After that, a solution in which an RNA aptamer whose 5'-terminal had a biotin was diluted into a concentration of 5 µM with HBS-P(+MgCl2) was dripped onto the region containing the detection part 13. Then, the chip was left still for 5 minutes and, after that, rinsed with HBS-P(+MgCl2). Here, at the time of formation of the reference electrode, this processing was not performed.
(8) A hemoglobin sample having been diluted into 11.6 µM with HBS-P(+MgCl2) was introduced through the passage 15 to the sensing element 3. Then, the amount of phase change occurring after 5 minutes of reaction was measured.

According to this, since a high strength linkage between an SA and a biotin was employed, the RNA aptamer was allowed to be immobilized with high stability and then detection of Hb was achieved.

### Fourth example (example that amino group was provided at terminal)

A procedure of immobilizing the RNA aptamer onto a mounting chip and then detecting hemoglobin is described below.

Procedures (1) to (3) were the same as the procedures (1) to (3) of the third example mentioned above.

In the subsequent procedures (4) to (6) described below, the aptamer was immobilized onto the immobilization membrane Au.
(4) A phosphate buffer solution in which polyethylene glycol (PEG) having an average molecular weight of 2000 has been dissolved was dripped onto the region containing the detection part 13. Then, the chip was left still for 5 minutes and, after that, rinsed with the phosphate buffer solution. Here, as for the terminals of PEG, one terminal had a thiol group and the other terminal had a carboxyl group.
(5) The phosphate buffer solution was substituted with water. Then, a mixture liquid of a 1-ethyl-3(3-dimethylaminopropyl)carbodiimide aqueous solution and an N-hydroxysuccinimide aqueous solution was dripped onto the region containing the detection part 13. Then, the chip was left still for 20 minutes and, after that, rinsed with water so that active esterification of carboxyl group located at a terminal of 3,3'-dithiodipropionic acid was performed.
(6) The water was removed. Then, a solution in which an RNA aptamer whose 5'-terminal had an amino group was diluted into a concentration of with 5 µM with HBS-N (manufactured by GE Healthcare) was dripped. Then, the chip was left still for 5 minutes and, after that, processed. Here, at the time of formation of the reference electrode, this processing was not performed.
(7) A hemoglobin sample having been diluted into 11.6 µM with HBS-P(+MgCl2) was introduced through the passage 15 to the sensing element 3. Then, the amount of phase change occurring after 5 minutes of reaction was measured.

According to this, at the time of immobilization of the RNA aptamer, non-specific binding to gold was suppressed so that the RNA aptamer was allowed to bind to a terminal of PEG in an excellent orientation and then detection of Hb was achieved.

### Fifth example (example that thiol group was provided at terminal)

A procedure of immobilizing the RNA aptamer onto a mounting chip and then detecting hemoglobin is described below.

Procedures (1) to (3) were the same as the procedures (1) to (3) of the third example mentioned above.

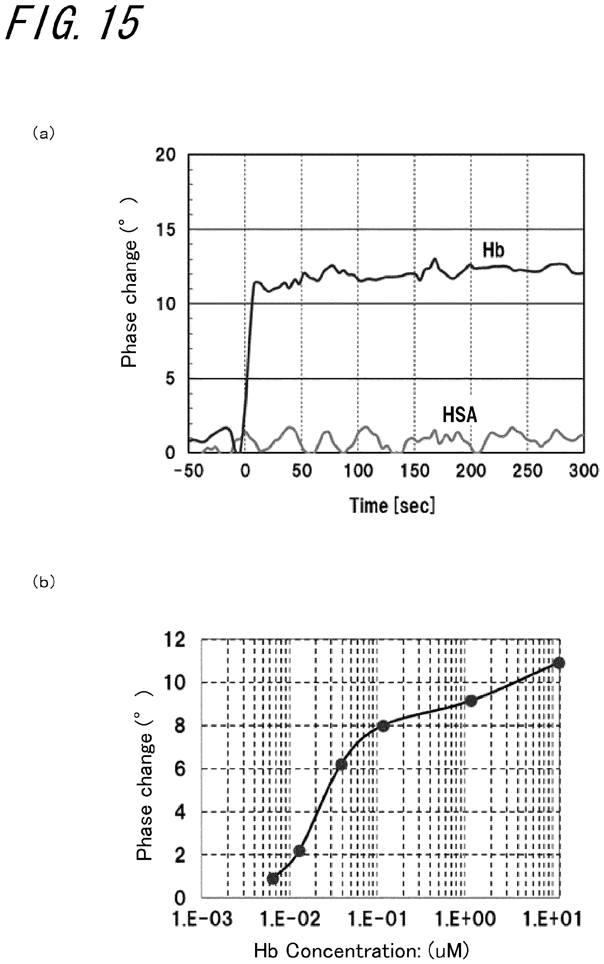

In the subsequent procedures (4) to (10) described below, the aptamer was immobilized onto the immobilization membrane Au.
(4) An RNA aptamer whose 5'-terminal had a thiol group was dripped onto the Au membrane on the detection side for 5 minutes. Then, the chip was left still. At that time, the RNA aptamer was dissolved in HBS-P(+MgCl2).
(5) A phosphate buffer solution in which polyethylene glycol (PEG) having an average molecular weight of 2000 has been dissolved was dripped onto the individual Au membranes on the detection side and on the reference side. Then, the chip was left still for 5 minutes. Here, as for the terminals of PEG, one terminal was a thiol group and the other terminal was a methoxy group.
(6) The above-mentioned procedure (5) was repeated twice.
(7) The region containing the detection part 13 was rinsed with 10-mM NaOH.
(8) A phosphate buffer solution in which polyethylene glycol (PEG) having an average molecular weight of 1000 has been dissolved was dripped onto the individual Au membranes on the detection side and on the reference side. Then, the chip was left still for 5 minutes. Here, as for the terminals of PEG, one terminal was a thiol group and the other terminal was a methoxy group.
(9) The above-mentioned procedure (8) was repeated three times.
(10) The region containing the detection part 13 was rinsed with 10-mM NaOH.
(11) A hemoglobin sample having been diluted into 11.6 µM with HBS-P(+MgCl2) was introduced through the passage 15 to the sensing element 3. Then, the amount of phase change occurring after 5 minutes of reaction was measured. Similarly, measurement was performed with employing human serum albumin (HSA) as a sample. These measurement results are shown in FIG. 15(a). Further, as for the hemoglobin sample, measurement was performed on samples of various concentrations. The measurement results are shown in FIG. 15(b).

FIG. 15(a) shows the results obtained so that each sample was introduced to the sensing element 3 at time point 0 second and then binding between the aptamer and the target was observed up to 300 seconds. The vertical axis indicates the amount of phase change caused by binding between the aptamer and the target.

According to this, it has been recognized that the RNA aptamer having a sequence of linkage order of UAUUAGGACCA can specifically bind to Hb and has a tendency not to bind to human serum albumin (HSA).

FIG. 15(b) shows a relationship between the RNA aptamer and the concentration of Hb serving as the sample. Here, for each sample concentration, the average of the values of amount of phase change from 180 seconds to 300 seconds posterior to the sample introduction was acquired and is shown.

According to this, it has been recognized that in the RNA aptamer, the ratio of binding to Hb increases with increasing Hb concentration.

The invention is not limited to the above-mentioned embodiment and may be implemented in various modes.

For example, the above-mentioned embodiment has been described for a case where the detection part 13 is constructed from a metal membrane and the RNA aptamer 130 immobilized on the surface of the metal membrane. However, in place of the metal membrane, the RNA aptamer 130 may be immobilized on the surface of a membrane having no electrical conductivity.

Further, the above-mentioned embodiment has been described for a case where the sensing element 3 is constructed from a surface acoustic element. However, the employable sensing element 3 is not limited to this. For example, a sensing element 3 may be employed in which an optical waveguide or the like is formed so that surface plasmon resonance may occur. In this case, for example, measurement is performed on a change in the light refractive index or the like in the detection part. Alternatively, a sensing element 3 may be employed in which a vibrator is formed on a piezoelectric plate of quartz or the like. In this case, for example, measurement is performed on a change in the oscillation frequency of the vibrator.

Further, as the sensing element 3, plural kinds of devices may be disposed on one chip. For example, an enzymatic electrode according to an enzymatic electrode method may be provided adjacent to the SAW element. In this case, in addition to the immunity method employing an antibody or an RNA aptamer, measurement by an enzymatic method can be performed. Thus, the number of items inspected at one time can be increased.

Further, the above-mentioned embodiment has been described for an example that one sensing element 3 alone is provided. Instead, a plurality of sensing elements 3 may be provided. In this case, the element accommodation recess 5 may be provided for each sensing element 3 or, alternatively, the element accommodation recess 5 having a length or width allowing the accommodation of all sensing elements 3 may be provided.

Further, the above-mentioned embodiment has been described for an example that the first cover member 1, the intermediate cover member 1A, and the second cover member 2 are in a separate construction from each other. However, employable configurations are not limited to this, and some of the members may be integrated together.

### Reference Signs List

1: First cover member
1A: Intermediate cover member
1Aa: First upstream part
θ1a: Contact angle
1Ab: First downstream part
θ1b: Contact angle
2: Second cover member
2a: Third substrate
2b: Fourth substrate
2ba: Second upstream part
θ2a: Contact angle
2bb: Second downstream part
θ2b: Contact angle
3: Sensing element
3a: Upstream region
θ3a: Contact angle
3b: Sensing region (detection part)
θ3b: Contact angle
3c: Downstream region
θ3c: Contact angle
4: Recess forming site
5: Element accommodation recess
6: Terminal
7: Wiring
9: Filling member
10: Substrate
11: First IDT electrode
12: Second IDT electrode
13(3b): Detection part
13a: Immobilization membrane (Immobilization member)
13b: Protective film
13c: Chain-like substance
130: RNA aptamer (Binding part)
S: Sequence
130a: First region
130a1: First base pair
130a11: First end
130a12: Second end
130aa: First sequence
130ab: Second sequence
130ax: First site
130ay: Second site
130b: Second region
130c: Terminal region
130c1: 3'-terminal
130c2: 5'-terminal
131a: Biotin
131b: Thiol group
131c: Amino group
132: First substance
133: Double strand
134: Streptavidin
135: Detection target (Hemoglobin)
14: Inflow part
15: Passage
15a: Upstream part
15b: Downstream part (Extension part)
18: Air release hole
19: First extraction electrode
19e: End part
20: Second extraction electrode
20e: End part
27: Lead wire (metal thin wire)
28: Insulating member
30: Liquid absorbing material
100, 101: Sensor

## Claims

1. An RNA aptamer, comprising:
a sequence of linkage order of UAUUAGGACCA,
in which an A denotes a nucleotide whose base is adenine, a G denotes a nucleotide whose base is guanine, a C denotes a nucleotide whose base is cytosine, and a U denotes a nucleotide whose base is uracil.

2. The RNA aptamer according to claim 1, wherein the U located at a first end of the sequence is positioned on a 5'-terminal side relative to the A located at a second end of the sequence.

3. The RNA aptamer according to claim 1 or 2, further comprising a first region containing the U and the A which are located at both ends of the sequence.

4. The RNA aptamer according to any one of claims 1 to 3, wherein the base of the U and the base of the A which are located at both ends of the sequence constitute a first base pair.

5. The RNA aptamer according to any one of claims 1 to 4, further comprising a plurality of nucleotides attached to at least one of the U and the A which are located at both ends of the sequence.

6. The RNA aptamer according to claim 5, wherein
the plurality of nucleotides comprises:
a first sequence composed of at least one nucleotide attached to the U located at the first end of the sequence; and
a second sequence composed of at least one nucleotide attached to the A located at the second end of the sequence.

7. The RNA aptamer according to claim 6, wherein the first region includes a first site where the first sequence and the second sequence constitute at least one base pair.

8. The RNA aptamer according to claim 6 or 7, wherein the first region further includes a second site where the first sequence and the second sequence do not constitute a base pair.

9. The RNA aptamer according to any one of claims 1 to 8, wherein at least one of the C and the U in the sequence contains a fluorine group provided at a 2'-position of the nucleotide.

10. The RNA aptamer according to any one of claims 1 to 9, further comprising a biotin located at a terminal thereof.

11. The RNA aptamer according to claim 10, further comprising a streptavidin attached to the biotin.

12. The RNA aptamer according to any one of claims 1 to 9, further comprising a thiol group located at a terminal.

13. The RNA aptamer according to claim 12, further comprising an Au attached to the thiol group.

14. The RNA aptamer according to any one of claims 1 to 9, further comprising an amino group located at a terminal.

15. The RNA aptamer according to claim 14, further comprising a first substance which forms an amide linkage with the amino group.

16. The RNA aptamer according to claim 15, wherein the first substance includes a polyethylene glycol.

17. A sensor, comprising:
a substrate; and
an RNA aptamer according to any one of claims 1 to 16, the RNA aptamer being immobilized to the substrate.

18. The sensor according to claim 17, further comprising an immobilization member which is located between the RNA aptamer and the substrate and is attached to at least one of the RNA aptamer and the substrate.
